# EUROPEAN PATENT APPLICATION

(11) **EP 4 234 683 A1**
(43) Date of publication of application: **30.08.2023**
(21) Application number: 21883286.3
(22) Date of filing: 21.10.2021
(51) Int. Cl.: C12N 5/071, C12N 5/00

(54) **METHOD FOR CONSTRUCTION OF ATOPIC DERMATITIS MODEL BY USING PLURIPOTENT STEM CELL-DERIVED SKIN ORGANOID**

(30) Priority: 21.10.2020 KR 20200136977; 20.10.2021 KR 20210140422
(71) Applicant: Kangstem Biotech Co., Ltd., Seoul 06179 (KR)
(72) Inventor: KANG, Kyung-Sun, Seoul 06338 (KR); JUNG, Song-Yi, Seoul 08745 (KR)
(74) Representative: Dehns
(86) International application number: PCT/KR2021/014810
(87) International publication number: WO 2022/086218

(57) **Abstract**

The present invention relates to a method for constructing an atopic dermatitis skin organoid from human pluripotent stem cells. It was observed that ALI-skin organoids of the present invention formed skin cells, appendages, neural cells, adipocytes, and the like in similar structures to those in the real skin and exhibited greatly improved follicle growth, compared to skin organoids constructed by existing culture methods. In addition, as a result of treatment with Staphylococcus aureus to construct an atopic dermatitis model, a good simulation was made of the characteristics of atopic dermatitis, such as damaged skin barriers, increased epithelium-derived cytokines and downregulated epidermal stem cell expression, causing Staphylococcus aureus colonies, and the like. Thus, the skin organoid of the present invention is expected to find advantageous applications as a real skin model and an atopic dermatitis model.

## Description

### [Technical Field]

The present invention relates to a method for constructing an atopic dermatitis model using a human pluripotent stem cell derived skin organoid, and the like.

The present application claims priority to and the benefit of Korean Patent Application Nos. 10-2020-0136977 and 10-2021-0140422 filed in the Korean Intellectual Property Office on October 21, 2020 and October 20, 2021, respectively, and all the contents disclosed in the specification and drawings of these applications are incorporated in the present application.

### [Background Art]

Human skin has the functions of maintaining body fluids, regulating body temperature, and protecting against external stress or external harmful bacteria, and plays an important role in mediating touch and pain. The skin is composed of the epidermal layer, the dermal layer and the subcutaneous layer, and appendages such as hair follicles and sebaceous glands, and neural cells are present in the skin. In particular, the interaction between the epidermal layer and the dermal layer is very important for the development of the skin and the formation of its appendages.

Currently, most studies on human skin development and disease have been conducted using animal experiments and *ex vivo* artificial skin models. However, animal experimental models differ from the human body in terms of genetic/biological characteristics, and artificial skin models often used for toxicity and drug screening include only the basal epidermis and dermis, which do not produce appendages such as sweat glands, hair follicles and melanocytes present in real skin. Thus, there is an increasing need for developing a skin model that more closely resemble real skin capable of simulating the complexity and function of *in vivo* skin tissue.

Recently, a method for culturing a 3D organoid capable of simulating the tissues and functions in living organs using human pluripotent stem cells has been developed, and the 3D organoid is a cultured embryoid body capable of reproducing the morphology and function of the tissue or organ of a real organ, and is also called an organ analog or pseudo organ. Recently, methods for culturing a skin organoid which resembles real skin, the skin organoid having various human skin cells, neural cells, and adipocytes, and having appendages, and the like formed therein, have been developed. However, since organoids formed by the skin organoid culture method of existing research (see Non-Patent Document 1) show a three-dimensional circular structure different from real skin, it is difficult to use the organoid as a model for drug toxicity efficacy experiments, skin transplantation, and atopic skin diseases.

Meanwhile, atopic dermatitis is a chronic/intractable/inflammatory skin disease that exhibits symptoms of dry skin lesions accompanied by erythema, itching, a rash, and cutaneous keratin. Atopic dermatitis affects 15 to 30% of children and 2 to 10% of adults in developed countries worldwide, and currently, 10% of the population and 20% of the children in Korea also suffer from atopic dermatitis. According to statistical data from the Health Insurance Review & Assessment Service, the number of patients with an atopic disease in Korea, who visit hospitals each year is close to 1 million, and the estimated number of patients is about 2 million according to the prevalence rate. It is known that the currently identified cause of the disease is caused by complex interactions such as filaggrin gene mutation, immunological factors, and skin barrier damage. Further, recent studies have suggested an increase in colonization of Staphylococcus aureus and a decrease in bacterial diversity (dysbiosis) on the skin as potential causes of atopic dermatitis. However, the exact disease mechanism and suitable therapeutic agents have not yet been clarified. In addition, the disease mechanism and drug screening research of atopic dermatitis are currently mainly based on research using two-dimensional culture methods using existing cell lines, *in vitro* artificial skin models, and animal models.

Therefore, there is a need for developing an improved skin organoid model, which has a structure more similar to real skin and can simulate the complexity and function of skin tissue *in vivo.*

### [Non-Patent Document]

(Non-Patent Document 1) The latest research and development trend of 3D skin model, SungJae Jang, BRIC View 2019-T21

### [Disclosure]

### [Technical Problem]

Thus, the present inventors constructed a three-dimensional air-liquid interface (ALI) organoid model (ALI-skin organoid) from human pluripotent stem cells by applying an air-liquid interface culture method in order to efficiently construct three-dimensionally layered skin organoids similar to real human skin, and developed an atopic dermatitis organoid model that enables the investigation of the mechanism of atopic skin diseases and disease factors by inoculating ALI-skin organoids with Staphylococcus aureus, and enables research on the effect of disease therapeutic agents through drug screening.

Therefore, an object of the present invention is to provide a method for constructing a skin organoid from human pluripotent stem cells, including the following steps:
(a) culturing a pluripotent stem cell-derived organoid in the presence of a Wnt agonist;
(b) culturing a culture product of Step (a) in a medium for skin organoid maturation for 40 days or more; and
(c) cutting a culture product of Step (b) and culturing the cut culture product at the air-liquid interface.

Another object of the present invention is to provide a method for constructing a skin organoid, the method including treating a skin organoid constructed by the above method with Staphylococcus aureus, in which the skin organoid is a skin simulation model of atopic dermatitis.

Still another object of the present invention is to provide a skin organoid constructed by the method, in which the skin organoid is a skin simulation model of atopic dermatitis.

Yet another object of the present invention is to provide a screening method for a therapeutic agent for atopic dermatitis, the method including treating the skin organoid with a candidate material for a therapeutic agent for atopic dermatitis.

However, technical problems to be solved by the present invention are not limited to the aforementioned problems, and other problems that are not mentioned may be clearly understood by those skilled in the art from the following description.

### [Technical Solution]

Therefore, to achieve the aforementioned objects of the present invention,
the present invention provides a method for constructing a skin organoid from human pluripotent stem cells, the method including the following steps:
(a) culturing a pluripotent stem cell-derived organoid in the presence of a Wnt agonist;
(b) culturing a culture product of Step (a) in a medium for skin organoid maturation for 40 days or more; and
(c) cutting a culture product of Step (b) and culturing the cut culture product at the air-liquid interface.

In an exemplary embodiment of the present invention, the Wnt agonist may be CHIR-99021, but is not limited thereto.

In another exemplary embodiment of the present invention, the Wnt agonist may be added on days 5 to 7 of culturing pluripotent stem cells, but is not limited thereto.

In still another exemplary embodiment of the present invention, the medium for skin organoid maturation may include one or more components selected from the group consisting of GlutaMax^{™}, 2-mercaptoethanol, B-27, N2, and normocin, but is not limited thereto.

In yet another exemplary embodiment of the present invention, the method may include the following steps before performing Step (a), but is not limited thereto:
(1) culturing an embryoid body of pluripotent stem cells;
(2) inducing the embryoid body to differentiate into non-neural ectoderm; and
(3) inducing the non-neural ectoderm to differentiate into cranial neural crest-like cells.

In yet another exemplary embodiment of the present invention, Step (1) may be performed in the presence of Y-27632, but is not limited thereto.

In yet another exemplary embodiment of the present invention, Step (2) may be performed in the presence of SB431542, FGF2, and BMP4, but is not limited thereto.

In yet another exemplary embodiment of the present invention, Step (3) may be performed in the presence of FGF2 and LDN193189, but is not limited thereto.

In yet another exemplary embodiment of the present invention, the culture of Step (a) may be performed for 5 to 14 days, but is not limited thereto.

In yet another exemplary embodiment of the present invention, the culture of Step (b) may be performed for 40 to 100 days, but is not limited thereto.

In yet another exemplary embodiment of the present invention, the culture of Step (c) may be performed for 3 to 4 weeks, but is not limited thereto.

In yet another exemplary embodiment of the present invention, the method may further include (d) culturing the culture product of Step (c) under dry conditions for 1 to 10 days, but is not limited thereto.

In yet another exemplary embodiment of the present invention, Step (d) may be for stratum corneum maturation of the skin organoid, but is not limited thereto.

In yet another exemplary embodiment of the present invention, in Step (c), the culture product of Step (b) may be cut into four uniform sizes and the cut culture product of Step (b) may be cultured such that the dermal layer and the epidermal layer on a collagen-coated transwell culture insert are exposed to the collagen side and air, respectively, but is not limited thereto.

In yet another exemplary embodiment of the present invention, the air-liquid interface culture of Step (c) may be performed in a medium for skin organoid maturation, but is not limited thereto.

In yet another exemplary embodiment of the present invention, the skin organoid may have suppressed cartilage formation, but is not limited thereto.

In yet another exemplary embodiment of the present invention, the skin organoid may have a diameter of 2 to 20 mm, but is not limited thereto.

In yet another exemplary embodiment of the present invention, the skin organoid may have a dermal layer and a subcutaneous fat layer located under the epidermal layer, but is not limited thereto.

In yet another exemplary embodiment of the present invention, the skin organoid may express one or more selected from the group consisting of Keratin 5 (KRT5, CK5), KRT10, KRT15, KRT17, loricrin, filaggrin, SRY-Box Transcription Factor 2 (SOX2), and melan-A, but is not limited thereto.

In yet another exemplary embodiment of the present invention, the pluripotent stem cells may be embryonic stem cells or induced pluripotent stem cells, but are not limited thereto.

Further, the present invention provides a method for constructing a skin organoid, the method including treating a skin organoid constructed by the above method with Staphylococcus aureus, in which the skin organoid is a skin simulation model of atopic dermatitis.

In addition, the present invention provides a skin organoid constructed by the method, in which the skin organoid is a skin simulation model of atopic dermatitis.

In an exemplary embodiment of the present invention, the skin organoid may have one or more features selected from the group consisting of the following features, but is not limited thereto:
increased cell death in the skin dermal layer;
downregulated expression of one or more selected from the group consisting of KRT5, KRT10, filaggrin, loricrin, and Claudin 4, which are skin epidermal barrier-related markers;
decreased expression of KRT or KRTAP, which is a keratinocyte differentiation gene;
increased expression of one or more selected from the group consisting of β-defensin, peptidoglycan recognition protein 2 (PGLYRP2), toll-like receptor 1 (TLR1), TLR2, galectin 9 (LGALS9), lipocalin 2 (LCN2) and S 100 calcium binding protein (S100A), which are antimicrobial peptide genes;
increased expression of one or more selected from the group consisting of CXC motif chemokine ligand (CXCL), C-C motif chemokine ligand (CCL), thymic stromal lymphopoietin (TSLP), interleukin-1 (IL-1), and tumor necrosis factor (TNF), which are epidermal or dermal cell-derived inflammatory cytokine genes;
downregulated expression of an epidermal stem cell marker KRT 15; and
colonization of Staphylococcus aureus.

Furthermore, the present invention provides a screening method for a therapeutic agent for atopic dermatitis, the method including treating the skin organoid with a candidate material for a therapeutic agent for atopic dermatitis.

Further, the present invention provides a use of the skin organoid for screening a therapeutic agent for atopic dermatitis.

In addition, the present invention provides a pharmaceutical composition for preventing or treating atopic dermatitis, comprising *Cutibacterium acnes* or a culture solution thereof as an active ingredient.

In an exemplary embodiment of the present invention, the composition may increase the expression of filaggrin, but is not limited thereto.

Furthermore, the present invention provides a food composition for preventing or ameliorating atopic dermatitis, comprising *Cutibacterium acnes* or a culture solution thereof as an active ingredient.

Further, the present invention provides a cosmetic composition for preventing or ameliorating atopic dermatitis, comprising *Cutibacterium acnes* or a culture solution thereof as an active ingredient.

In addition, the present invention provides a method for preventing or treating atopic dermatitis, the method including administering a pharmaceutical composition comprising *Cutibacterium acnes* or a culture solution thereof as an active ingredient to a subject in need.

Furthermore, the present invention provides a use of a pharmaceutical composition comprising *Cutibacterium acnes* or a culture solution thereof as an active ingredient for preventing or treating atopic dermatitis.

Further, the present invention provides a use of *Cutibacterium acnes* or a culture solution thereof for producing a drug for preventing or treating atopic dermatitis.

### [Advantageous Effects]

The present inventors confirmed that treatment with a Wnt agonist on day 6 not only remarkably increased the size of organoid, but also suppressed the formation of cartilage, which is an unnecessary component of skin organoids. It was observed that ALI-skin organoids of the present invention formed skin cells, appendages, neural cells, adipocytes, and the like in similar structures to those in real skin and exhibited greatly improved follicle growth, compared to skin organoids constructed by existing culture methods, and it was confirmed that organoids are formed in similar structures to those in real skin. In addition, as a result of treatment with *Staphylococcus aureus* to construct an atopic dermatitis model, a good simulation of the characteristics of atopic dermatitis, such as a damaged skin barrier, increased epithelium-derived cytokines, downregulated skin barrier function-related gene expression, downregulated keratinocyte differentiation-related gene expression, increased antimicrobial peptide gene expression, increased epidermal and dermal cell-derived cytokine gene expression, downregulated epidermal stem cell expression, causing Staphylococcus aureus colonization, and the like was made. Thus, the skin organoid of the present invention is expected to find advantageous applications as a real skin model and an atopic dermatitis model.

Furthermore, using the atopic dermatitis model of the present invention, it was confirmed that *Cutibacterium acnes,* which is a beneficial human commensal bacterium as a microbiome therapeutic agent, diminished the skin barrier disruption due to infection with *Staphylococcus aureus,* and provided a protective mechanism against colonization of *Staphylococcus aureus.* Therefore, *Cutibacterium acnes* is expected to be useful for the treatment of atopic dermatitis through significant preventive and therapeutic effects against Staphylococcus aureus-mediated skin barrier disruption and cell damage.

### [Description of Drawings]

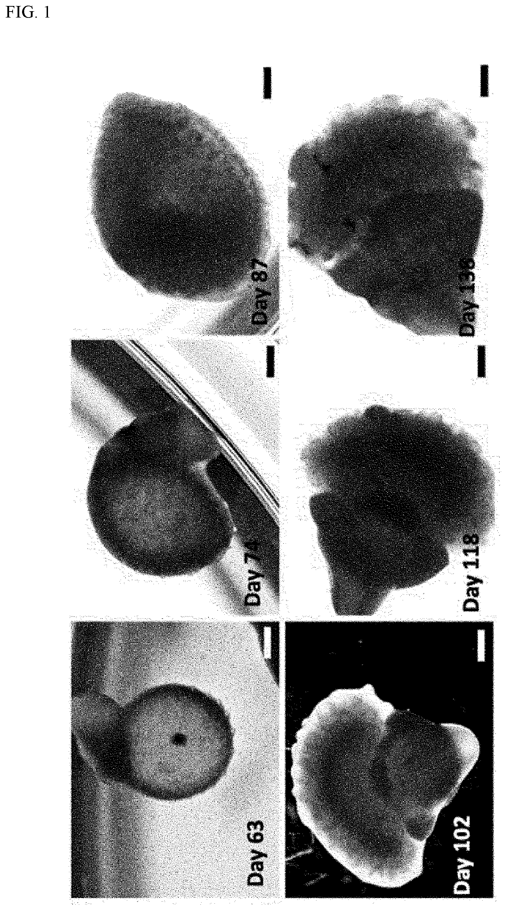

FIG. 1 illustrates the timelines (Day 63 to Day 138) of skin organoid formation using a human-induced pluripotent stem cell line based on the existing method and photographs of each stage. Scale bars = 500 µm.

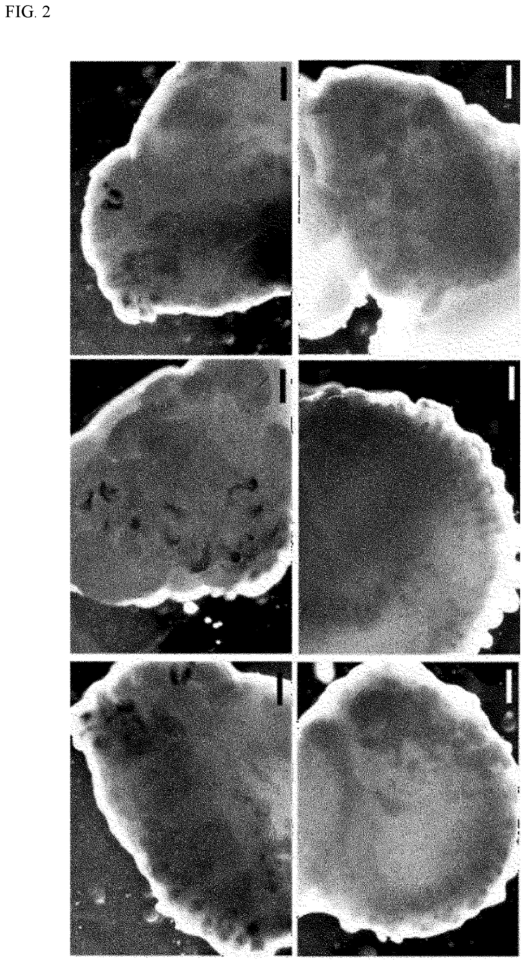

FIG. 2 is a set of skin organoid photographs of a skin organoid constructed using the existing method on day 140, confirming the aspect including pigmented hair follicles (upper panel) and albino hair follicles (lower panel). Scale bars = 500 µm.

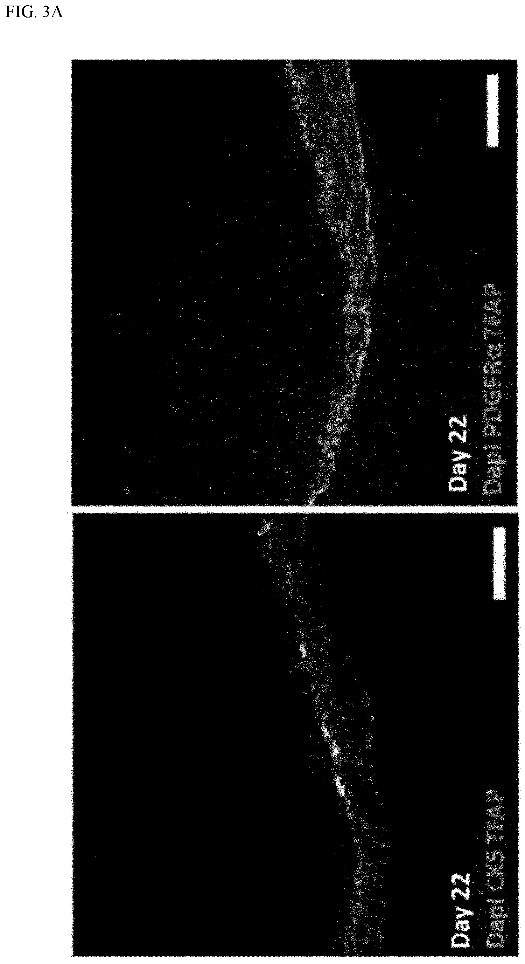

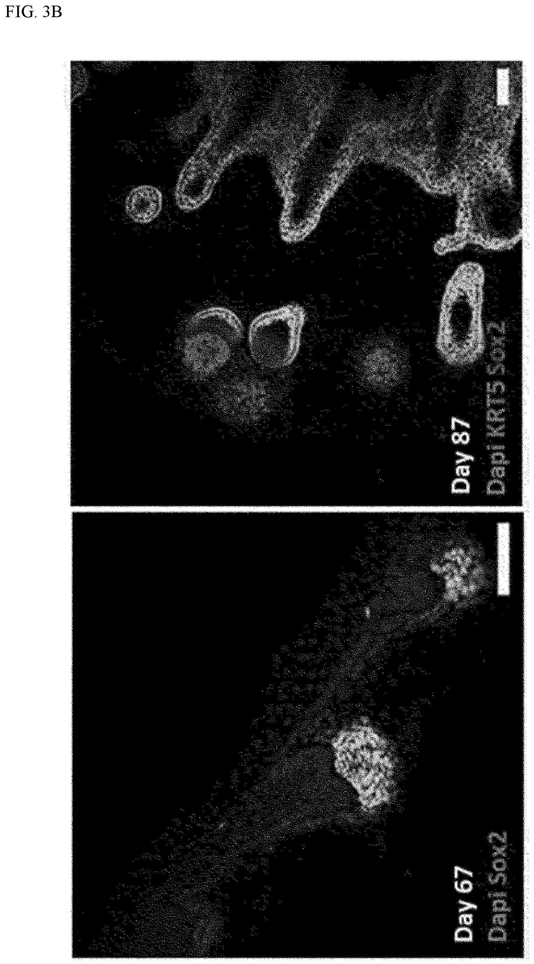

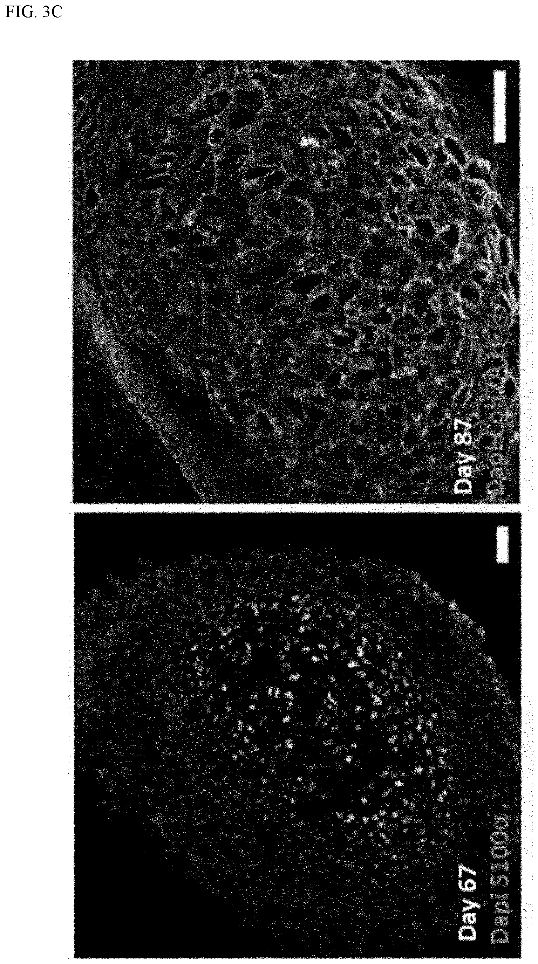

FIGS. 3A to 3C illustrate photographs of differentiation marker fluorescence staining of skin organoids constructed using the existing method. FIG. 3A is a staining photograph (left) of epidermal progenitor cell markers (TFAP+KRT5+) and a staining photograph (right) of dermal progenitor cells (TFAP+PDGFRα+) of skin organoids on day 22, FIG. 3B is a staining photograph of a skin hair follicle dermal papilla cell expression marker (Sox2) on day 67 (left) and day 83 (right), and FIG. 3C illustrates staining photographs of hyaline cartilage chondrocyte differentiation markers (S100α; left and Coll2A1; right). Scale bars = 50 µm.

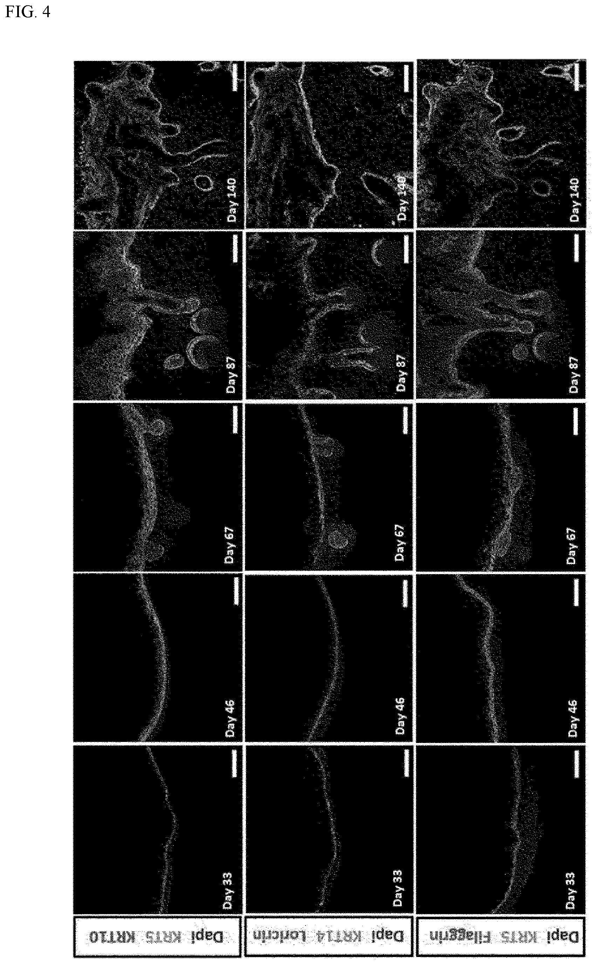

FIG. 4 illustrates the timeline of epidermal layer formation in skin organoids constructed using the existing method. FIG. 4 illustrates immunohistochemical analysis results for KRT5 and KRT14 (basement membrane markers), KRT10 (stratum spinosum marker), loricrin and filaggrin (stratum corneum markers). Scale bars = 50 µm.

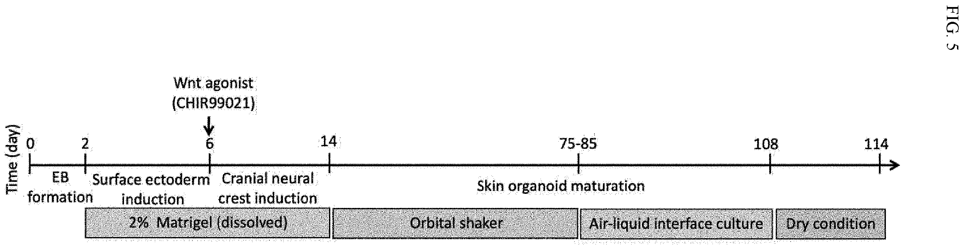

FIG. 5 illustrates the method for culturing a three-dimensional ALI-skin organoid from the human pluripotent stem cells of the present invention by adding Wnt signaling pathway activation (0.5 to 8 µM Wnt agonist (CHIR-99021)).

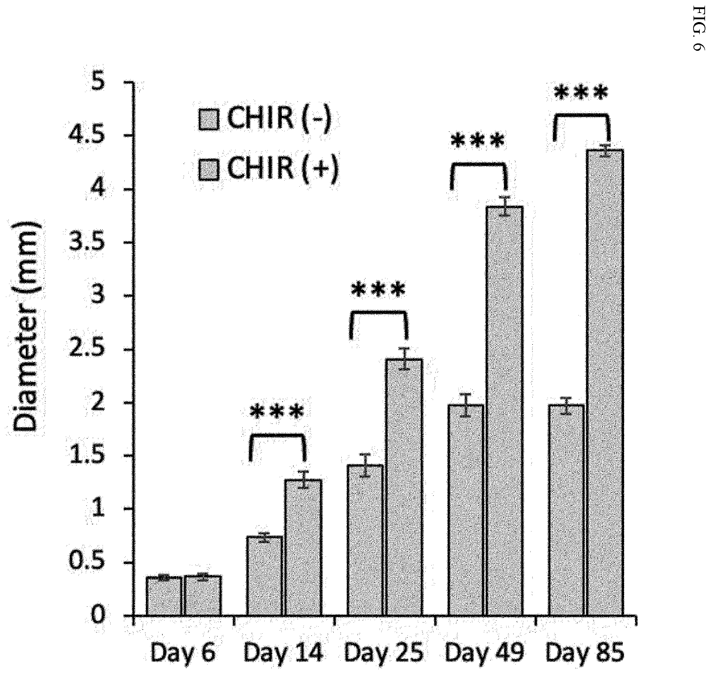

FIG. 6 illustrates the results of quantifying the diameter (mm) of a skin organoid based on images of the skin organoid on days 6, 14, 25, 49 and 85. CHIR (-): CHIR-99021 not added, CHIR (+): CHIR-99021 added. Mean ± SEM; n=9; *** represents P<0.0005.

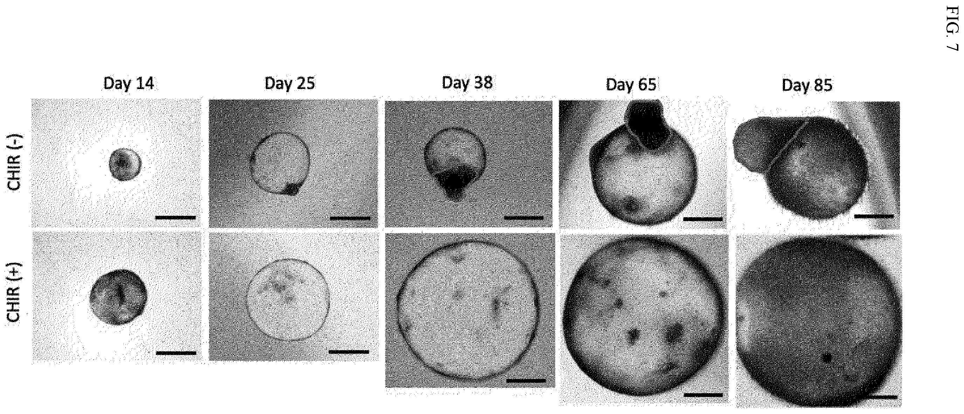

FIG. 7 illustrates photographs of induced pluripotent stem cell-derived skin organoids on day 14, 25, 38, 65, or 85. CHIR (-): CHIR-99021 not added, CHIR (+): CHIR-99021 added. Red circles in the photographs of skin organoids of CHIR (-) on days 38, 65, and 85 indicate cartilage structures. Scale bars = 1 mm.

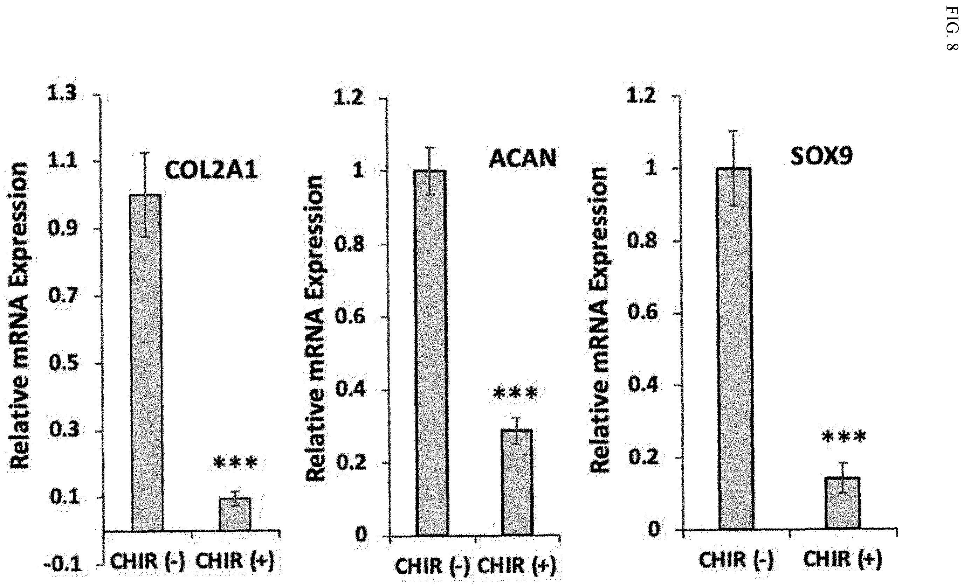

FIG. 8 is a set of results of analyzing the gene expression levels of cartilage expression markers COL2A1, ACAN, and SOX9 in induced pluripotent stem cell-derived skin organoids on day 69 by real-time PCR. CHIR (-): CHIR-99021 not added, CHIR (+): CHIR-99021 added. n=3; *** represents P<0.0005

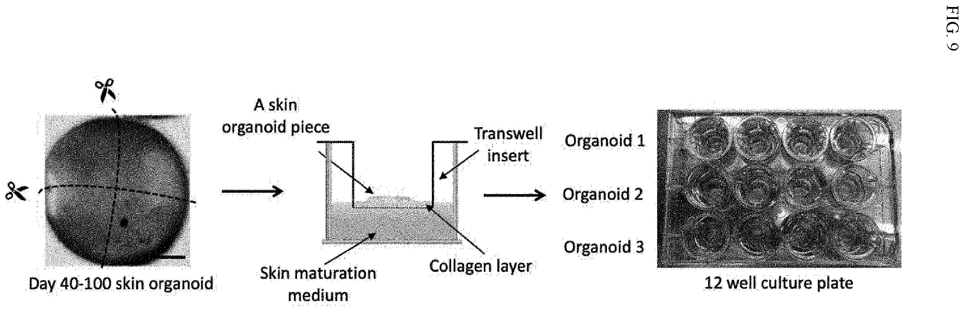

FIG. 9 illustrates a method for constructing a three-dimensional air-liquid interface-skin organoid model (ALI-skin organoid) from human induced pluripotent stem cells in which the air-liquid interface (ALI) culture method is applied.

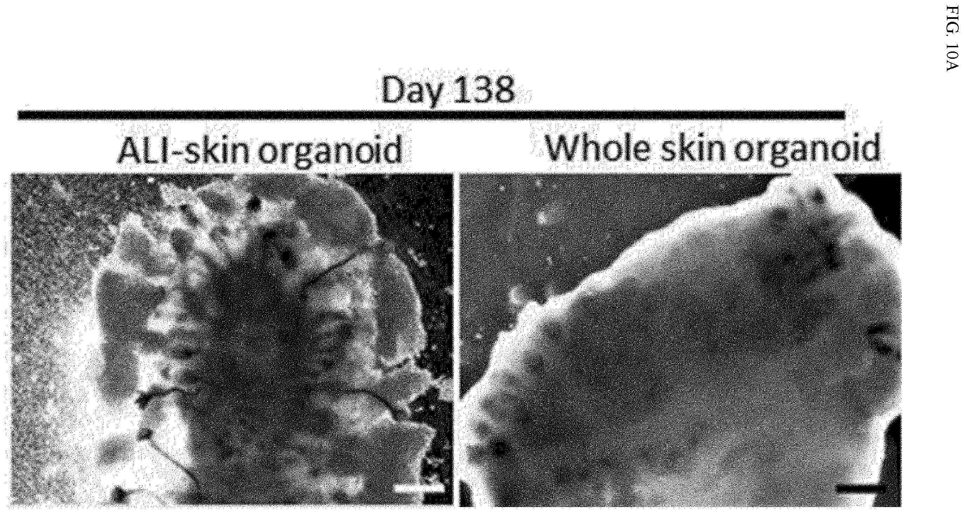

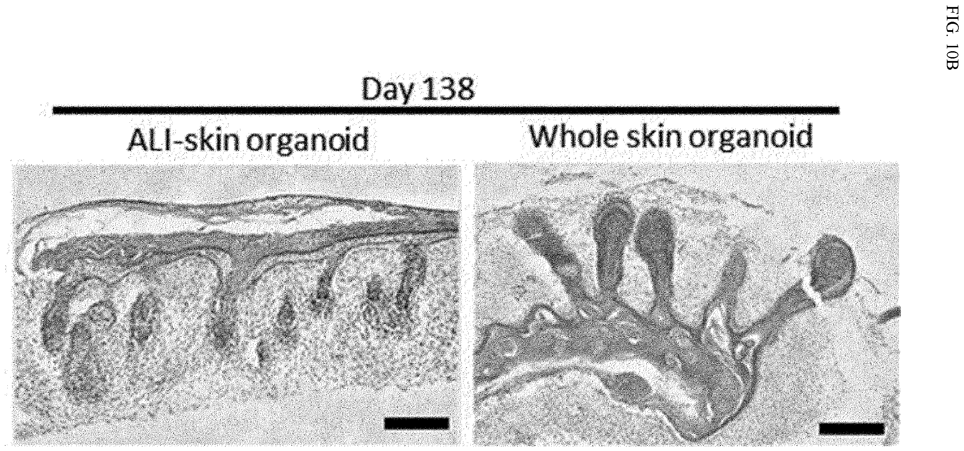

FIGS. 10A and 10B illustrate the results of comparing an ALI-skin organoid with a skin organoid cultured according to the existing protocol (whole skin organoid), FIG. 10A illustrates a representative photograph comparing the ALI-organic skin and the existing organic skin on day 138, and FIG. 10B illustrates the results of hematoxylin & eosin (H&E) tissue staining. Scale bars = 500 µm.

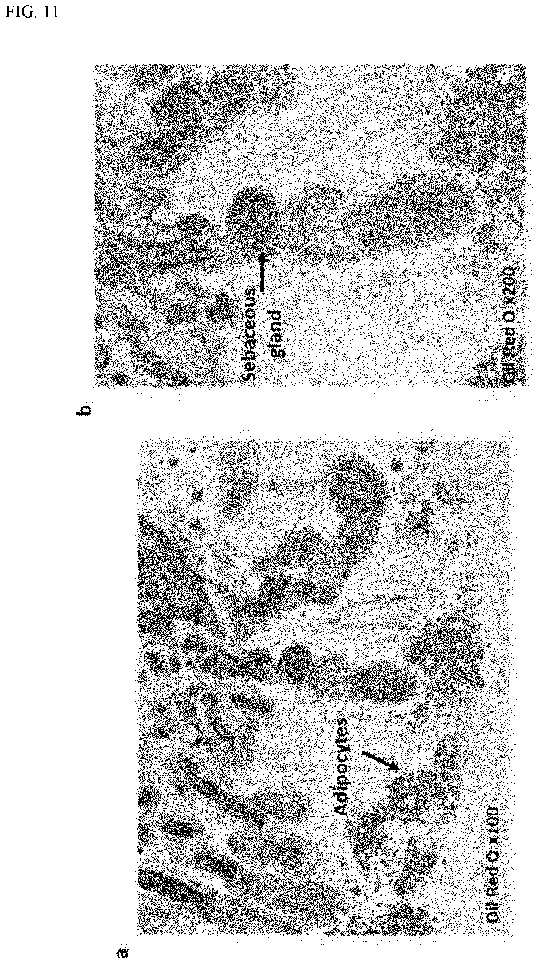

FIG. 11 illustrates the results of staining subcutaneous fat of ALI-skin organoids and sebaceous glands developed around hair follicles with Oil Red O. ×100 (left) and x200 (right).

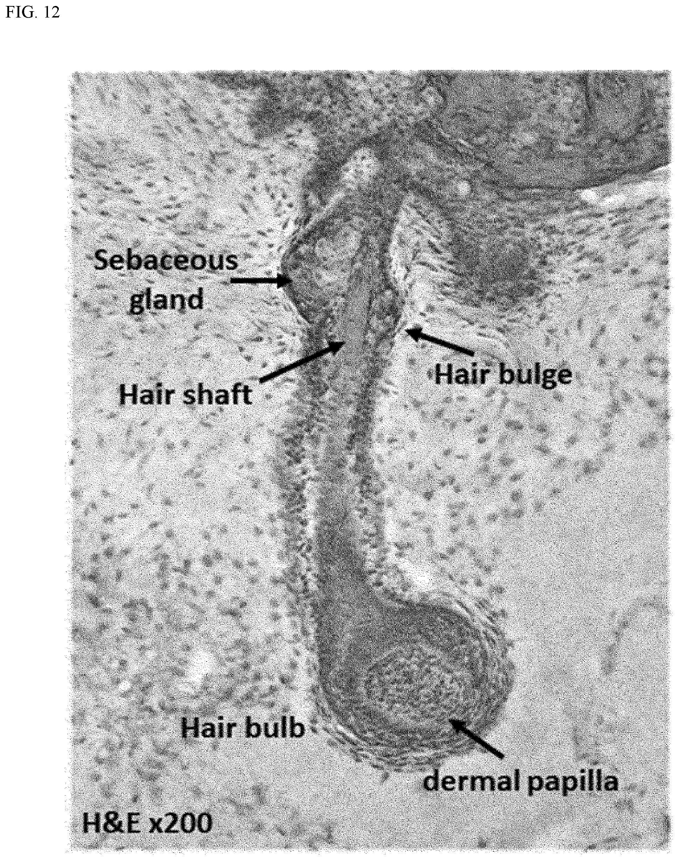

FIG. 12 illustrates a photograph (x200) of H&E staining of hair follicles in the ALI-skin organoid on day 119 in total. (Skin organoid culture day 68 + ALI culture day 51)

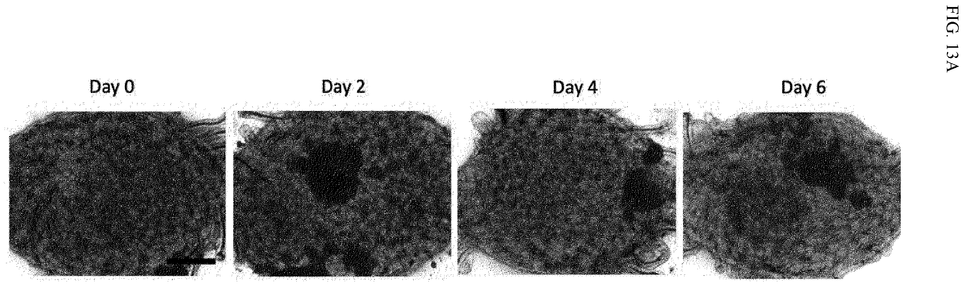

FIG. 13A is a photograph of the ALI-skin organoid on day 114 in total, in which the skin organoid was cultured for 85 days, ALI-cultured under 100% humidity conditions for 3 weeks, and then ALI-cultured for 0 days, 2 days, 4 days, or 6 days to mature skin keratinocytes under dry conditions. Scale bars = 500 µm.

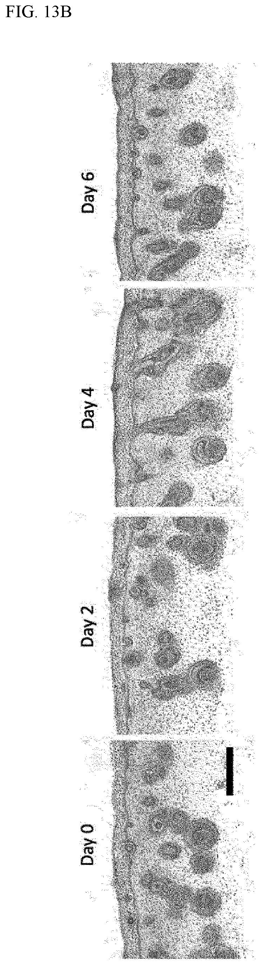

FIG. 13B illustrates hematoxylin & eosin (H&E) tissue staining results for the ALI-skin organoid on day 114 in total. The skin organoid was cultured for 85 days, ALI-cultured under 100% humidity conditions for 3 weeks, and then ALI-cultured for 0 days, 2 days, 4 days, or 6 days to mature skin keratinocytes under dry conditions. Scale bars = 500 µm.

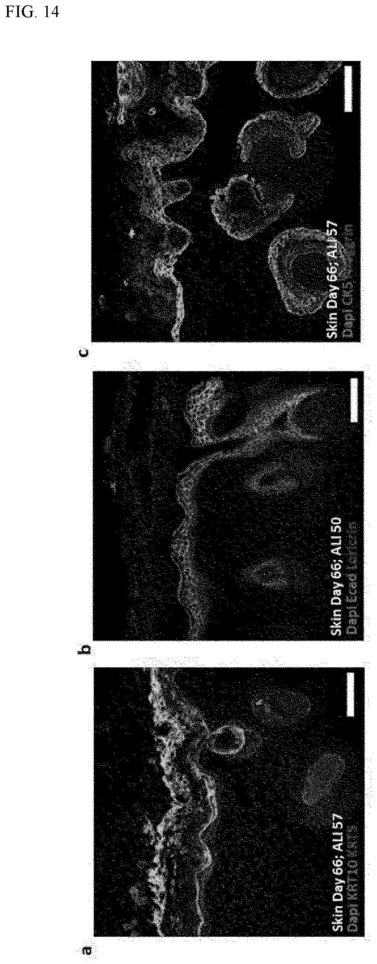

FIG. 14 illustrates fluorescence staining photographs of the skin epidermal layer structure-related markers of the ALI-skin organoid. (A) is a staining photograph of an ALI-skin organoid basement membrane marker (KRT5) and a stratum spinosum marker (KRT10), (B) is a staining photograph of an ALI-skin organoid stratum corneum marker (loricrin), and (C) is a staining photograph of an ALI-skin organoid stratum corneum marker (filaggrin). Scale bars = 50 µm.

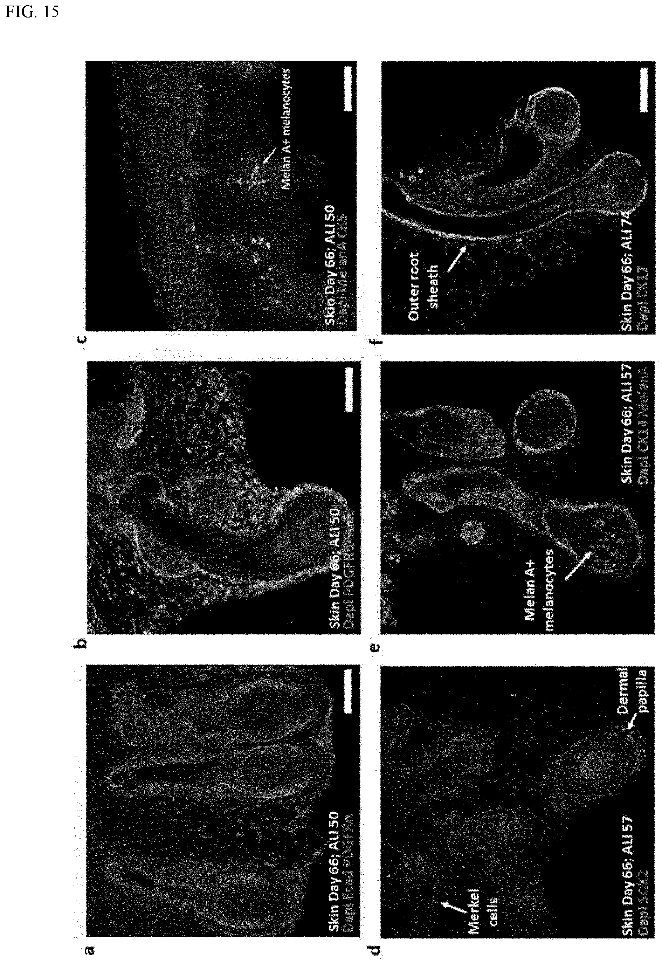

FIG. 15 illustrates fluorescence staining photographs of ALI-skin organoid-hair follicle structure-related markers. (A) is a staining photograph of an ALI-skin organoid epidermal cell marker (E-cadherin) and a dermal cell marker (PDGGR), (B) is a staining photograph of an ALI-skin organoid hair bulge area marker (KRT15), (C) is a staining photograph of an ALI-skin organoid epidermal layer melanocyte marker (melan-A), (D) is a staining photograph of an ALI-skin organoid hair follicle dermal papilla cell marker (Sox2) and a Merkel cell marker (Sox2), (E) is a staining photograph of an ALI-skin organoid hair follicle melanocyte marker (melan-A), and (F) is a staining photograph of an ALI-skin organoid outer root sheath marker (KRT17). Scale bars = 50 µm.

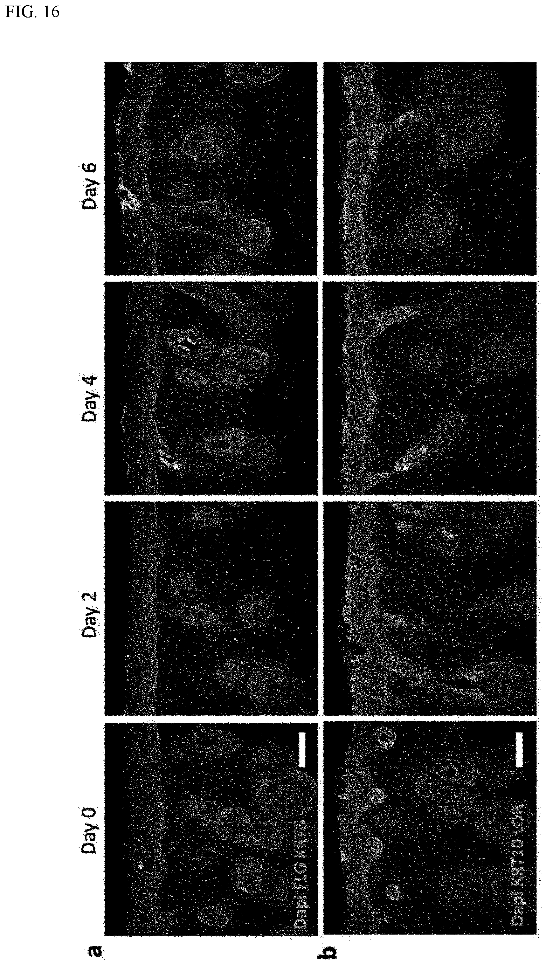

FIG. 16 illustrates fluorescence staining photographs of skin epidermal layer structure-related markers of skin organoids, in which the skin organoid was cultured for 85 days, ALI-cultured under 100% humidity conditions for 3 weeks, and then ALI-cultured for 0 days, 2 days, 4 days, or 6 days to mature skin keratinocytes under dry conditions. 'a' is a set of staining photographs of an ALI-skin organoid basement membrane marker (KRT5) and a stratum corneum marker (filaggrin), and 'b' is a set of photographs of an ALI-skin organoid stratum corneum marker (loricrin) and a stratum spinosum marker (KRT10). Scale bars = 50 µm.

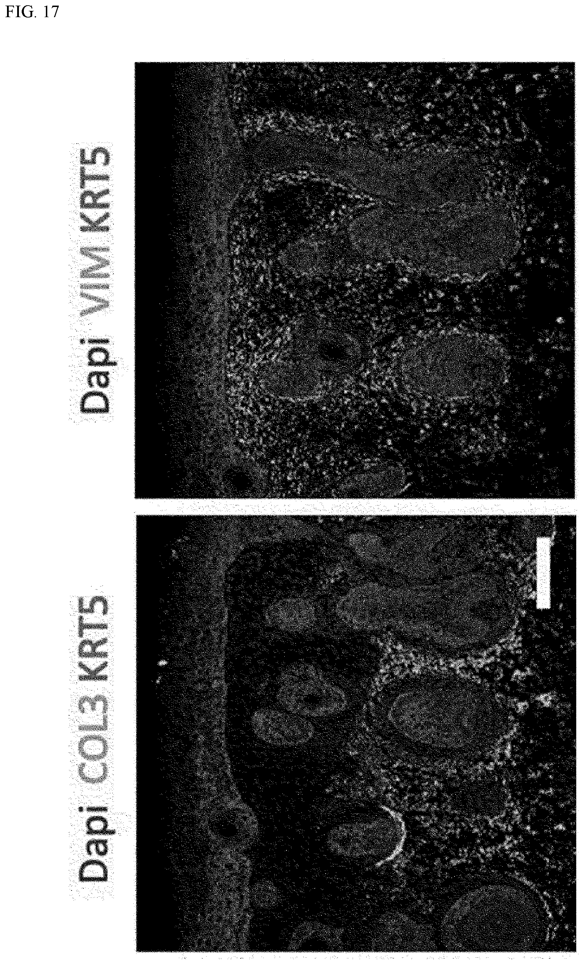

FIG. 17 illustrates fluorescence staining photographs of skin dermal layer structure-related markers of skin organoids, in which the skin organoid was cultured for 85 days, ALI-cultured under 100% humidity conditions for 3 weeks, and then ALI-cultured for 0 days, 2 days, 4 days, or 6 days to mature skin keratinocytes under dry conditions. The photograph is a staining photograph of ALI-skin organoid basement membrane marker (KRT5; red), dermal layer marker collagen 3 (COL3; green; left) and vimentin marker (VIM; green; right). Scale bars = 50 µm.

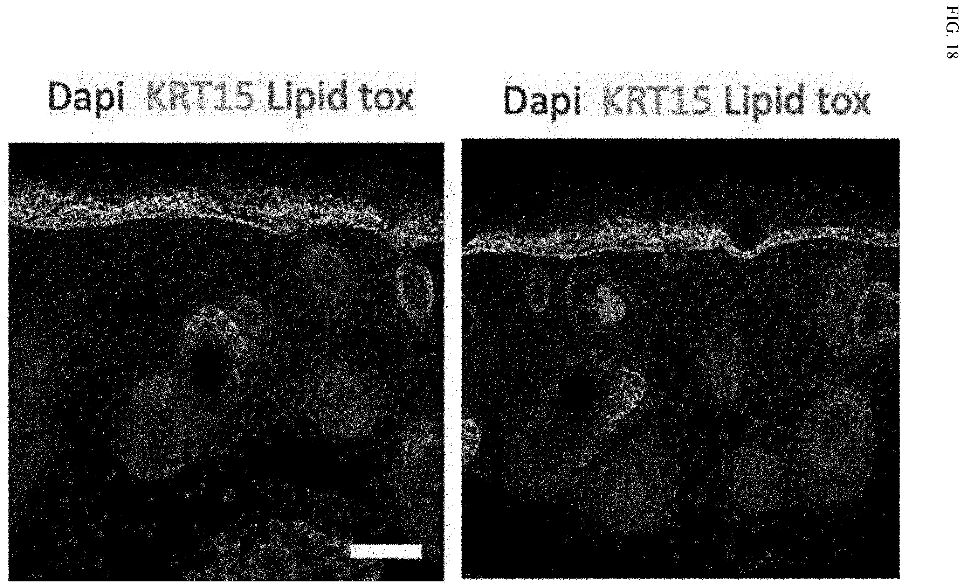

FIG. 18 illustrates the results of staining the subcutaneous fat (Lipid tox; red; left) and sebaceous glands (Lipid tox; red; right) developed around hair follicles of skin organoids cultured for 85 days, ALI-cultured under 100% humidity conditions, and ALI-cultured for 0 days, 2 days, 4 days, or 6 days to mature skin keratinocytes under dry conditions. Scale bars = 50 µm.

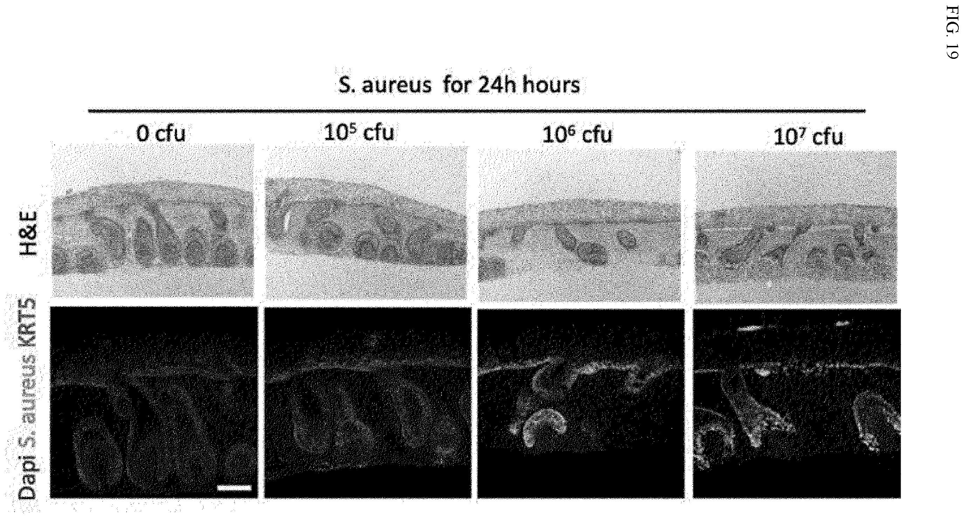

FIG. 19 illustrates photographs of modeling atopic dermatitis by inoculating and infecting the surface of ALI-skin organoids with *Staphylococcus aureus.* Specifically, FIG. 19 illustrates (top) ALI-skin organoid H&E staining photographs and (bottom) staining photographs of *Staphylococcus aureus* (green) and a basal epithelial layer marker (KRT5, red), 24 hours after inoculating *Staphylococcus aureus* (ATCC 29213; 0 CFU(PBS only), 1×10⁵ CFU, 1×10⁶ CFU or 1×10⁷ CFU) onto the surface of the ALI-skin organoid. Scale bars = 50 µm.

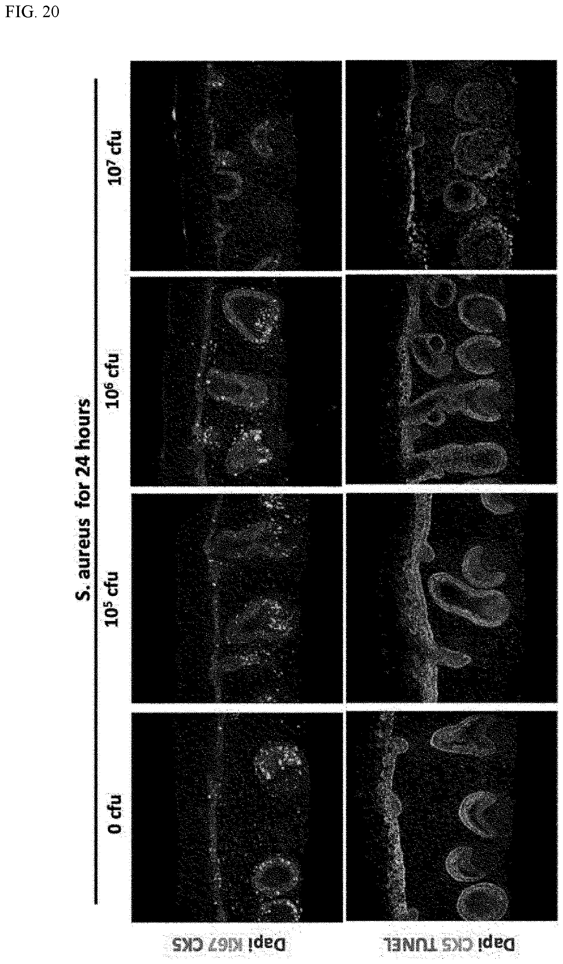

FIG. 20 illustrates the modeling of atopic dermatitis by infecting ALI-skin organoids with *Staphylococcus aureus,* and illustrates (top) staining photographs of a cell proliferation marker (Ki67; green) and a basal epithelial layer marker (KRT5; red), and (bottom) staining photographs of a cell death marker (TUNEL, red) and a basal epithelial layer marker (KRT5; green), 24 hours after inoculating *Staphylococcus aureus* onto the surface of the ALI-skin organoid. Scale bars = 50 µm.

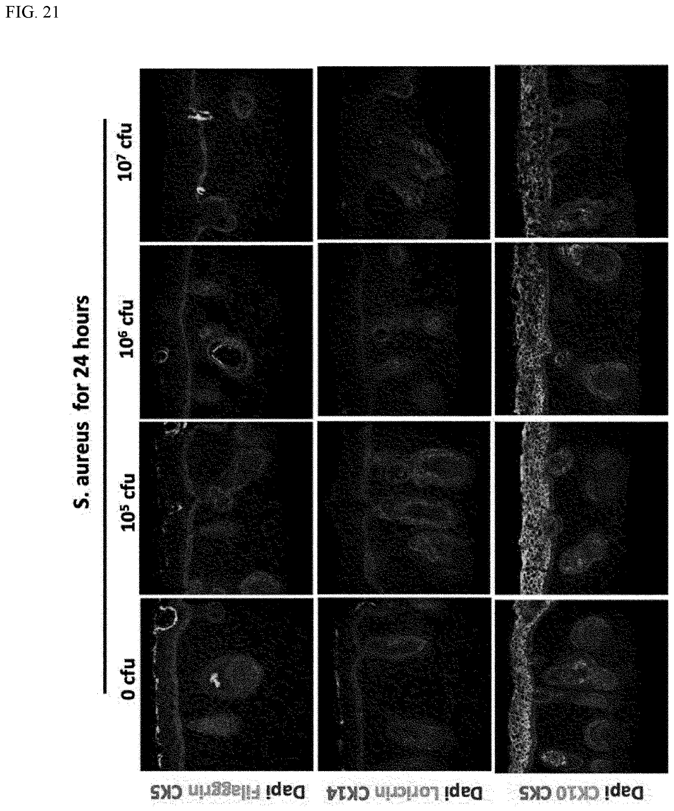

FIG. 21 illustrates the modeling of atopic dermatitis by infecting ALI-skin organoids with *Staphylococcus aureus,* and illustrates (top) staining photographs of a stratum corneum marker (filaggrin; green) and a basal epithelial layer marker (KRT5; red), (middle) staining photographs of a stratum corneum marker (loricrin; green) and a basal epithelial layer marker (KRT14; red), and (bottom) staining photographs of a stratum spinosum marker (KRT 10; green) and a basal epithelial layer marker (KRT5; red), 24 hours after inoculating *Staphylococcus aureus* onto the surface of the ALI-skin organoid. Scale bars = 50 µm.

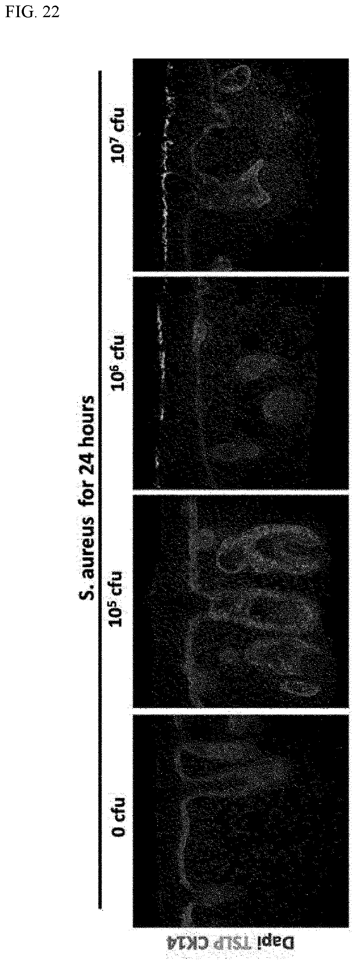

FIG. 22 illustrates the modeling of atopic dermatitis by infecting ALI-skin organoids with *Staphylococcus aureus,* and illustrates the results of confirming skin epithelial cell cytokine expression through fluorescence staining, 24 hours after inoculating *Staphylococcus aureus* onto the surface of the ALI-skin organoid. FIG. 22 illustrates staining photographs of *Staphylococcus aureus-*infected ALI-skin organoid epithelial cell cytokine (TSLP; green) and basal epithelial layer (KRT14; red) markers. Scale bars = 50 µm.

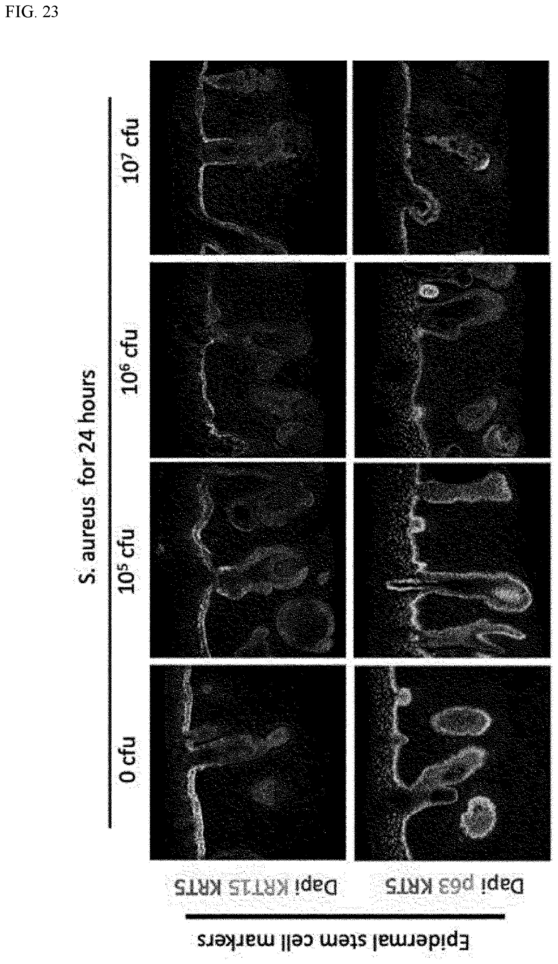

FIG. 23 illustrates the modeling of atopic dermatitis by infecting ALI-skin organoids with *Staphylococcus aureus,* and illustrates the results of confirming a decrease in expression of skin epithelial stem cells through fluorescence staining, 24 hours after inoculating *Staphylococcus aureus* onto the surface of the ALI-skin organoid. FIG. 23 illustrates (top) staining photographs of *Staphylococcus aureus-*infected ALI-skin organoid epidermal stem cell (KRT15; green) and basal epithelial layer (KRT5; red) markers, and (bottom) an epidermal stem cell marker (p63; green) and a basal epithelial layer marker (KRT5; red). Scale bars = 50 µm.

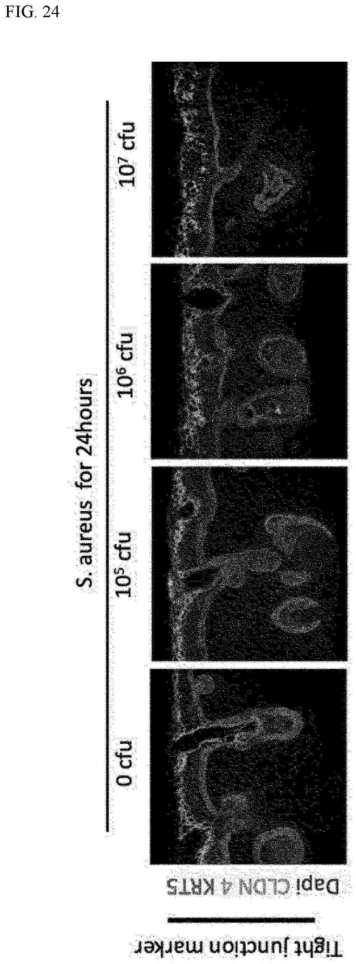

FIG. 24 illustrates the modeling of atopic dermatitis by infecting ALI-skin organoids with *Staphylococcus aureus,* and illustrates the results of confirming skin epithelial cell cytokine expression through fluorescence staining, 24 hours after inoculating *Staphylococcus aureus* onto the surface of the ALI-skin organoid. FIG. 24 illustrates staining photographs of an aggregate protein (Claudin 4 (CLDN4); green), which is a tight junctional protein marker, and a basal epithelial layer marker (KRT5; red). Scale bars = 50 µm.

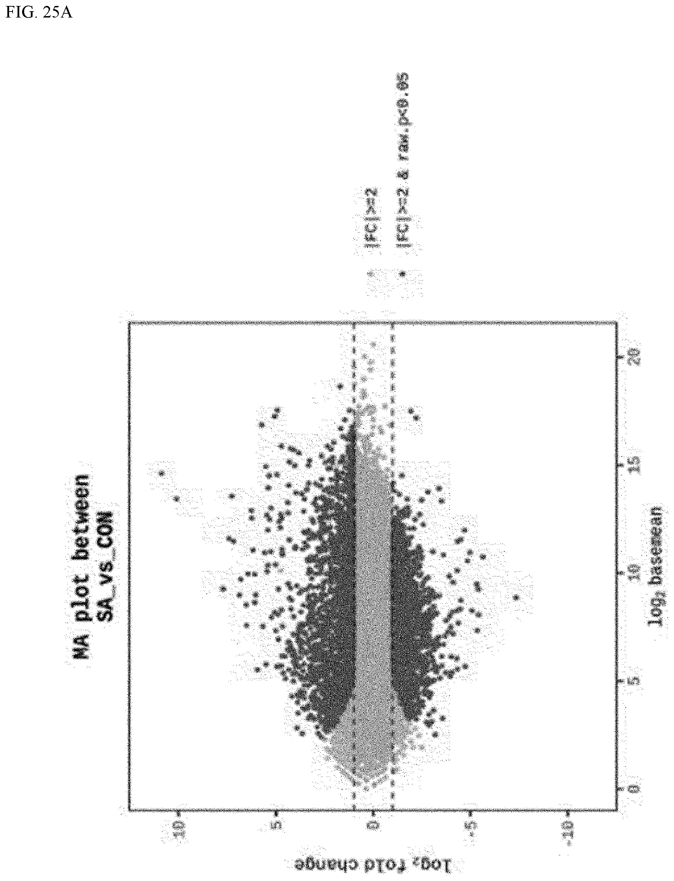

FIGS. 25A to 25F illustrate the modeling of atopic dermatitis by infecting ALI-skin organoids with *Staphylococcus aureus,* and illustrate the results of confirming a change in gene expression by an RNA sequencing analysis method, 18 hours after inoculating *Staphylococcus aureus* onto the surface of the ALI-skin organoid. It was confirmed that significant differences in the expression of numerous genes are shown between biological replicates between ALI-skin organoids treated with *Staphylococcus aureus* and untreated controls.
FIG. 25A illustrates a volcano plot representation of differentially expressed genes (DEG; dark blue).
FIG. 25B is illustrated by visualization through a heat map depicting the differentially expressed genes. Out of 4,255 differentially expressed genes (DEGs), 2,162 genes were highly expressed and 2,093 genes were underexpressed by the inoculation of *S. aureus* (adjusted p-value < 0.05). Red indicates the control and blue indicates the ALI-skin organoids treated with *Staphylococcus aureus.*
FIG. 25C illustrates the results of comparing the expression of skin barrier function-related genes between S. *aureus-infected* ALI-skin organoids and untreated controls by RNA sequencing analysis.
FIG. 25D illustrates the results of comparing the expression of keratinocyte differentiation-related genes between S. *aureus-infected* ALI-skin organoids and untreated controls by RNA sequencing analysis.
FIG. 25E illustrates the results of comparing the expression of antimicrobial peptide (AMP)-related genes between S. *aureus-infected* ALI-skin organoids and untreated controls by RNA sequencing analysis.
FIG. 25F illustrates the results of comparing the expression of inflammatory cytokine-related genes between S. *aureus-infected* ALI-skin organoids and untreated controls by RNA sequencing analysis.
FIG. 26A illustrates the ALI-skin organoid culture method and *Cutibacterium acnes* pretreatment and *Staphylococcus aureus* inoculation method.
FIG. 26B illustrates the results of analyzing immunohistochemical staining of filaggrin (green) and KRT5 (red) according to treatment with *Cutibacterium acnes.* Scale bars = 50 µm.

### [Modes of the Invention]

The present invention provides a method for constructing a skin organoid from human pluripotent stem cells, the method including the following steps:
(a) culturing a pluripotent stem cell-derived organoid in the presence of a Wnt agonist;
(b) culturing a culture product of Step (a) in a medium for skin organoid maturation for 40 days or more; and
(c) cutting a culture product of Step (b) and culturing the cut culture product at the air-liquid interface.

Hereinafter, the present invention will be described in detail.

As used herein, the term "organoid" refers to a three-dimensional collection of one or more cell types that mimic(s) the superficial appearance or actual structure or function of a tissue or organ. Further, the organoid may similarly reproduce the physiological functions of the human body, and by constructing an organ analog from tissues of a patient, disease modeling based on the patient's genetic information, drug screening through repeated testing, and the like may be performed.

As used herein, "organoid culture" includes all actions that enable an organoid to be produced or maintained. For example, cells or cells isolated from specific tissues may differentiate into tissue or organ cells with specific functions, and/or organoids may survive, grow, or proliferate.

In the present invention, pluripotent stem cells are commonly referred to as stem cells that can differentiate into almost all types of cells that constitute endoderm, mesoderm and ectoderm. In the present invention, the pluripotent stem cells may be embryonic stem cells or induced pluripotent stem cells, but are not limited thereto.

As used herein, the term "embryonic stem cells (ESCs)" refers to cells which are obtained by extracting an inner cell mass from embryos in the blastula stage just before implantation of a fertilized egg into the mother's uterus and culturing the inner cell mass ex vivo and have pluripotency capable of differentiating into all cells of an animal, and preferably are human-derived human embryonic stem cells.

As used herein, the term "induced pluripotent stem cells (iPSCs, dedifferentiated stem cells)" refers to stem cells which are created by establishing undifferentiated stem cells with differentiation ability similar to embryonic stem cells using a dedifferentiation technique in somatic cells of an animal, and have differentiation ability comparable to ESCs.

As used herein, the term "embryoid body" refers to a mass of spherical stem cell-derived cells produced in a suspension culture state, and is used as a precursor in most differentiation-inducing processes to secure tissue-specific differentiated cells by having the ability to potentially differentiate into endoderm, mesoderm, and ectoderm.

In the present invention, the Wnt agonist may be CHIR-99021, but is not limited thereto.

In the present invention, the Wnt agonist may be added on days 5 to 7 of culturing induced pluripotent stem cells, but is not limited thereto.

In the present invention, the medium for skin organoid maturation may include one or more components selected from the group consisting of GlutaMax^{™}, 2-mercaptoethanol, B-27, N2, and normocin, but is not limited thereto. As used herein, the term "medium for skin organoid maturation" refers to a medium required for culturing pluripotent stem cells or pluripotent stem cell-derived organoids in the form of skin organoids. In an exemplary embodiment, the medium for skin organoid maturation includes 1X GlutaMax^{™}, 0.5X B-27 minus vitamin 1 A, 0.5X N2, a supplement, 0.1 mM 2-mercaptoethanol and 100 µg/ml normocin in an Advanced DMEM/F12 and Neurobasal medium at a ratio of 1:1.

In the present invention, the medium for skin organoid maturation may be a mixed medium of the Advanced DMEM/F12 and Neurobasal medium, but is not limited thereto.

In the present invention, air-liquid interface culture (ALI culture) may culture cells in a partially open culture vessel or a culture vessel partially filled with a medium, but is not limited thereto. The air-liquid interface culture may expose the surface of cells or organoids to air.

In the present invention, the air-liquid interface culture may be performed on a collagen-coated plate, but is not limited thereto.

In the present invention, the method may include the following steps before performing Step (a), but is not limited thereto:
(1) culturing an embryoid body of pluripotent stem cells;
(2) inducing the embryoid body to differentiate into non-neural ectoderm; and
(3) inducing the non-neural ectoderm to differentiate into cranial neural crest-like cells.

In the present invention, Step (1) may be performed in the presence of Y-27632, but is not limited thereto.

In the present invention, Step (2) may be performed in the presence of SB431542, FGF2, and BMP4, but is not limited thereto.

In the present invention, Step (3) may be performed in the presence of FGF2 and LDN193189, but is not limited thereto.

In the present invention, the culture of Step (a) may be performed for 5 to 14 days, but is not limited thereto. For example, the culture of Step (a) may be performed for 5 days to 13 days, 5 days to 12 days, 5 days to 11 days, 5 days to 9 days, 6 days to 13 days, 6 days to 12 days, 6 days to 11 days, 6 days to 10 days, 6 days to 9 days, 7 days to 13 days, 7 days to 12 days, 7 days to 11 days, 7 days to 10 days, 7 days to 9 days, or about 8 days.

In the present invention, the culture of Step (b) may be performed for 40 to 100 days, but is not limited thereto. For example, the culture of Step (b) may be performed for 40 days to 95 days, 40 days to 90 days, 45 days to 95 days, 45 days to 90 days, 45 days to 80 days, 50 days to 80 days, 40 days to 75 days, 40 days to 70 days, 55 days to 75 days, 58 days to 75 days, 59 days to 74 days, 60 days to 72 days, or 61 days to 71 days.

In the present invention, Step (c) may be a step of cutting the culture product of Step (b) and culturing the cut culture product of Step (b) at the air-liquid interface. In an exemplary embodiment of the present invention, after cutting the culture product of Step (b) into 4 equal parts with scissors, each cut culture product was spread on a 12-well insert plate and cultured by an ALI culture method for 3 to 4 weeks in a 100% humidity condition incubator, and then cultured for 2 to 6 days under dry conditions (0% humidity condition incubator) for maturation of a stratified epithelial layer.

In the present invention, Step (c) may be for cutting the culture product of Step (b) into four uniform sizes, and
culturing the cut culture product of Step (b) such that the dermal layer and the epidermal layer on a collagen-coated transwell culture insert are exposed to the collagen side and air, respectively, but is not limited thereto.

Therefore, in the present invention, the method may further include (d) culturing the culture product of Step (c) under dry conditions for 1 to 10 days, but is not limited thereto. Step (d) may be performed for 1 to 10 days, 1 to 9 days, 1 to 8 days, 1 to 7 days, 1 to 6 days, 2 to 10 days, 2 to 9 days, 2 to 8 days, 2 to 7 days, 2 to 6 days, 3 to 10 days, 3 to 9 days, 3 to 8 days, 3 to 7 days, 3 to 6 days, 4 to 10 days, 4 to 9 days, 4 to 8 days, 4 to 7 days, or 4 to 6 days, but is not limited thereto.

Step (d) may be for stratum corneum maturation of skin organoids, but is not limited thereto.

In the present invention, the culture of Step (b) may be performed for 3 weeks to 4 weeks, but is not limited thereto.

In the present invention, the air-liquid interface culture of Step (c) may be performed in a medium for skin organoid maturation, but is not limited thereto.

The culture duration of the present invention may be adjusted in consideration of the amount and type of tissue, the type of medium used, the size, amount and characteristics of a desired organoid, and the like.

In the present invention, the skin organoid may have suppressed cartilage formation, but is not limited thereto.

In the present invention, the skin organoid may have a subcutaneous fat layer located under the dermal layer, but is not limited thereto.

In the present invention, the skin organoid may express one or more selected from the group consisting of Keratin 5 (KRT5), KRT10, KRT15, KRT17, loricrin, filaggrin, SRY-Box Transcription Factor 2 (SOX2), melan-A, collagen 3 (COL3) and vimentin (VMT), but is not limited thereto.

Further, the present invention provides a method for constructing a skin organoid, the method including treating a skin organoid constructed by the above method with Staphylococcus aureus, in which the skin organoid is a skin simulation model of atopic dermatitis.

Hereinafter, the method of the present invention will be described in detail, and descriptions overlapping with the above-described method for constructing a skin organoid will be omitted.

In addition, the present invention provides a skin organoid constructed by the method, in which the skin organoid is a skin simulation model of atopic dermatitis.

In the present specification, the skin organoid is characterized by having a flat shape rather than a spherical shape, but is not limited thereto.

In the present specification, the skin organoid may have a diameter of 2 to 20 mm, but is not limited thereto. In an exemplary embodiment of the present invention, it was confirmed that the skin organoid of the present invention has a larger diameter than organoids cultured by existing culture methods.

In the present specification, atopic dermatitis is a chronic inflammatory skin disease in which eczema lesion repeatedly recur in specific areas, accompanied by severe itching and overall dry skin, shows a genetic predisposition, and is characterized by the allergic march (progression from eczema to allergic diseases such as food allergies, allergic asthma, and allergic rhinitis). The pathogenesis of atopic dermatitis is largely divided into the pathogenesis associated with skin barriers and the pathogenesis associated with abnormal immune responses, and it has been reported that mutations in the filaggrin gene appear in about 40% of patients with atopic dermatitis, and more commonly appear when severe symptoms appear or persist into adulthood. The mechanism that causes atopic dermatitis is that external allergens pass more easily through the stratum corneum and sensitize the body in a person with congenitally damaged skin barriers.

In the present invention, the skin organoid may have one or more features selected from the group consisting of the following features, but is not limited thereto:
increased cell death in the skin dermal layer;
downregulated expression of one or more selected from the group consisting of KRT5, KRT10, filaggrin, loricrin, and Claudin 4, which are skin epidermal barrier-related markers;
decreased expression of KRT or KRTAP, which is a keratinocyte differentiation gene;
increased expression of one or more selected from the group consisting of β-defensin, peptidoglycan recognition protein 2 (PGLYRP2), toll-like receptor 1 (TLR1), TLR2, galectin 9 (LGALS9), lipocalin 2 (LCN2) and S 100 calcium binding protein (S 100A), which are antimicrobial peptide genes;
increased expression of one or more selected from the group consisting of CXC motif chemokine ligand (CXCL), C-C motif chemokine ligand (CCL), thymic stromal lymphopoietin (TSLP), interleukin-1 (IL-1), and tumor necrosis factor (TNF), which are epidermal or dermal cell-derived inflammatory cytokine genes;
downregulated expression of an epidermal stem cell marker KRT 15; and
colonization of Staphylococcus aureus.

In the present specification, filaggrin is one of various structural proteins expressed by keratinocytes during the differentiation stage, is involved in securing differentiation from the basal layer to the stratum corneum of the epidermis, and is used as a major indicator of skin moisture retention and skin membrane function, as it may also constitute a main component of a natural moisturizing factor (NMF), which is essential for retaining moisture in skin tissue.

In the present specification, loricrin is one of the skin barrier proteins expressed by keratinocytes during the differentiation stage.

In the present specification, thymic stromal lymphopoietin (TSLP) is a cytokine having a structure similar to that of IL-7, and is expressed in skin keratinocytes, epithelial cells, smooth muscle cells, pulmonary fibroblasts, and the like. TSLP activates dendritic cells to differentiate helper T cells into Th2 cells, and the differentiation of Th2 cells is important in inducing symptoms of atopic dermatitis.

As used herein, *Staphylococcus aureus* refers to a pathogenic bacterium that increases in atopic patients and exacerbates atopic symptoms.

In the present specification, *Staphylococcus aureus* may be treated at a concentration of 1×10¹ to 1×10²⁰ CFU, 1×10¹ to 1×10¹⁸ CFU, 1×10¹ to 1×10¹⁵ CFU, 1×10¹ to 1×10¹⁰ CFU, 1×10¹ to 1×10⁸ CFU, 1×10¹ to 1×10⁷ CFU, 1×10³ to 1×10²⁰ CFU, 1×10³ to 1×10¹⁸ CFU, 1×10³ to 1×10¹⁵ CFU, 1×10³ to 1×10¹¹ CFU, 1×10³ to 1×10⁹ CFU, 1×10³ to 1×10⁷ CFU, 1×10⁵ to 1×10²⁰ CFU, 1×10⁵ to 1×10¹⁵ CFU, 1×10⁵ to 1 ×10¹⁰ CFU, or 1×10⁵ to 1×10⁷ CFU, but is not limited thereto.

The skin organoid may be used *in vivo* to treat a subject. The present invention includes a method of treating a skin disease in a subject. As disclosed in the present invention, the method in one aspect includes the amelioration, treatment or alleviation of symptoms of a skin disease in a subject in need thereof. The method also includes administering a skin organoid in an amount effective to treat the skin disease of the subject.

The method of treating a subject may additionally include administration of a skin organoid or transplantation of the skin organoid, and in this case, as a result of administration or transplantation, the symptoms of the skin disease are ameliorated, treated or alleviated in the subject. The amelioration, treatment or alleviation may be any change in the skin disease or symptoms of the skin disease detectable using natural senses or artificial devices.

Skin diseases which can be treated using the method of the present invention include diseases or conditions manifested on the skin, for example, but not limited to, acne, atopic dermatitis, skin boil, furuncle, urticaria, psoriasis, seborrheic dermatitis, contact dermatitis, dyschromia, bacterial dermatitis, fungal dermatitis, eczema, edema, exanthema subitum, folliculitis, keratosis pilaris, warts, actinic keratosis, milium, xeroderma, hidradenitis, keloid disease, allergic dermatitis, cutaneous abscesses, neurodermatitis, and the like.

The skin organoid of the present invention may be included in an amount of 1 pg to 30 g w/v% in a pharmaceutical composition, but is not limited thereto.

The subject in the present invention is not limited as long as it is any vertebrate animal, but may be specifically applied to a human, a mouse, a rat, a guinea pig, a rabbit, a monkey, a pig, a horse, a cow, a sheep, an antelope, a dog and a cat, and may be preferably a human.

In the present invention, the "administration" refers to administration of the pharmaceutical composition of the present invention to a patient by any appropriate method, and for the route of administration of the composition of the present invention, the composition of the present invention may be administered via various oral or parenteral routes, as long as the composition of the present invention may reach a target tissue.

In the present invention, the "prevention" refers to all actions that delay skin diseases by administering the composition according to the present invention, the "treatment" refers to all actions that ameliorate or beneficially change skin disease symptoms by administration of the pharmaceutical composition according to the present invention, and the "amelioration" refers to all actions that reduce parameters associated with skin diseases by administration of the composition according to the present invention, for example, the severity of symptoms, by administration of the composition according to the present invention.

In the present invention, the pharmaceutical composition may further include an appropriate carrier, an appropriate excipient, and an appropriate diluent, which are typically used to prepare a pharmaceutical composition.

In the present invention, the "carrier" is also called a vehicle, and refers to a compound that facilitates the addition of a protein or peptide into a cell or tissue, and for example, dimethyl sulfoxide (DMSO) is a typically used carrier that facilitates the introduction of many organic materials into the cells or tissues of organisms.

In the present invention a "diluent" is defined as a compound diluted in water, which not only stabilizes the biologically active form of a protein or peptide of interest, but also dissolves the protein or peptide. A salt dissolved in a buffer solution is used as a diluent in the art. A typically used buffer solution is phosphate-buffered saline because it mimics the salt conditions of human body fluids. Since buffer salts can control the pH of a solution at low concentrations, buffer diluents rarely alter the biological activity of a compound. As used herein, azelaic acid-containing compounds may be administered to human patients either as such, or as pharmaceutical compositions along with other ingredients, as in combination therapy, or mixed with suitable carriers and excipients.

In addition, the pharmaceutical composition according to the present invention can be formulated in the form of an external preparation such as a powder, a granule, a tablet, a capsule, a suspension, an emulsion, a syrup, and an aerosol, and a sterile injection solution by typical methods, respectively, and examples of the carrier, excipient and diluent that may be included in the composition include lactose, dextrose, sucrose, oligosaccharide, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. When the pharmaceutical composition is prepared, the pharmaceutical composition is prepared using a diluent or excipient, such as a filler, an extender, a binder, a wetting agent, a disintegrant, and a surfactant, which are commonly used. A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with the compound. Further, in addition to a simple excipient, lubricants such as magnesium stearate and talc are also used. A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, an aromatic, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerogelatin, and the like.

The pharmaceutical composition of the present invention may be administered orally or parenterally, preferably parenterally, and in the case of parenteral administration, the composition may be administered by intramuscular injection, intravenous injection, subcutaneous injection, intraperitoneal injection, topical administration, transdermal administration or the like.

A suitable dose of the pharmaceutical composition of the present invention may vary depending on factors, such as formulation method, administration method, age, body weight, sex or disease condition of the patient, diet, administration time, administration route, excretion rate and response sensitivity.

The pharmaceutical composition of the present invention may be prepared in the form of a unit-dose or by being contained in a large capacity container by being formulated using a pharmaceutically acceptable carrier and/or excipient according to a method that can be readily implemented by a person with ordinary skill in the art to which the present invention pertains. In this case, a dosage form may also be in the form of a solution in an oil or aqueous medium, a suspension or in the form of an emulsion, an extract, a powder, a granule, a tablet or a capsule, and the pharmaceutical composition of the present invention may additionally include a dispersant or a stabilizer.

Furthermore, the present invention provides a screening method for a therapeutic agent for atopic dermatitis, the method including treating the skin organoid with a candidate material for a therapeutic agent for atopic dermatitis.

Further, the present invention provides a use of the skin organoid for screening a therapeutic agent for atopic dermatitis.

In addition, the present invention provides a pharmaceutical composition for preventing or treating atopic dermatitis, including *Cutibacterium acnes* or a culture solution thereof as an active ingredient.

In the present invention, the composition may increase the expression of filaggrin, but is not limited thereto.

Furthermore, the present invention provides a food composition for preventing or ameliorating atopic dermatitis, including *Cutibacterium acnes* or a culture solution thereof as an active ingredient.

Further, the present invention provides a cosmetic composition for preventing or ameliorating atopic dermatitis, including *Cutibacterium acnes* or a culture solution thereof as an active ingredient.

In addition, the present invention provides a method for preventing or treating atopic dermatitis, the method including administering a pharmaceutical composition including *Cutibacterium acnes* or a culture solution thereof as an active ingredient to a subject in need.

Furthermore, the present invention provides a use of a pharmaceutical composition including *Cutibacterium acnes* or a culture solution thereof as an active ingredient for preventing or treating atopic dermatitis.

Further, the present invention provides a use of Cutibacterium acnes or a culture solution thereof for producing a drug for preventing or treating atopic dermatitis.

In the present invention, *Cutibacterium acnes* is an anaerobic, Gram-positive bacterium, which is also named Propionibacterium acnes, and corresponds to the normal bacterial flora of human skin and mucosal tissue surfaces. *Propionibacterium acnes,* which is an anaerobic microorganism, grows inside hair follicles.

As used herein, the term 'culture' refers to all actions that are performed for growing microorganisms under appropriately artificially controlled environmental conditions, and is a concept including 'fermentation' in the present invention.

The bacterial cells of the *'Cutibacterium acnes'* include not only live cells obtained from the culture medium, but also any processed forms of lactic acid bacteria known to those skilled in the art, and include, for example, bacterial lysate products, dried products, frozen products, and the like, but are not limited thereto. In addition, the term 'culture solution' is a meaning including `fermentation liquid', and includes a culture solution itself cultured in a liquid medium, and processed products derived from a culture solution such as a filtrate (centrifuged supernatant) in which a strain is removed by filtering or centrifuging the above culture solution.

The content of the *Cutibacterium acnes* or a culture solution thereof in the composition of the present invention can be appropriately adjusted according to the symptoms of the disease, the degree of progression of the symptoms, the condition of the patient, and the like, and may be, for example, 0.0001 to 99.9 wt%, or 0.001 to 50 wt%, but is not limited thereto. The content ratio is a value based on a dry amount from which the solvent is removed.

In the present invention, the description on the pharmaceutical composition is described above.

When the *Cutibacterium acnes* or a culture solution thereof of the present invention is used as a food additive, the *Cutibacterium acnes* or a culture solution thereof may be added as it is or used with another food or other food ingredients, and may be appropriately used according to a typical method. The amount of active ingredient mixed may be suitably determined according to the purpose of use (prevention, health or therapeutic treatment). In general, when a food or beverage is prepared, the *Cutibacterium acnes* or a culture solution thereof of the present invention or the culture thereof may be added in an amount of 15 wt% or less, or 10 wt% or less based on the raw materials. However, in the case of long-term intake for the purpose of health and hygiene or for the purpose of controlling health, the amount may be less than the above range, and the effective ingredient may be used in an amount above the above range due to no problem in terms of safety.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not very important, but is generally selected within a range of 0.01 to 0.20 part by weight per 100 parts by weight of the composition of the present invention.

A formulation of the cosmetic composition according to the present invention may be in the form of a skin lotion, skin softener, skin toner, astringent, lotion, milk lotion, moisturizing lotion, nourishing lotion, massage cream, nourishing cream, mist, moisturizing cream, hand cream, hand lotion, foundation, essence, nourishing essence, pack, soap, cleansing foam, cleansing lotion, cleansing cream, cleansing oil, cleansing balm, body lotion or body cleanser.

The cosmetic composition of the present invention may further include a composition selected from the group consisting of water-soluble vitamins, oil-soluble vitamins, polymer peptides, polymeric polysaccharides, and sphingolipids.

Any water-soluble vitamin may be used as long as it can be incorporated into cosmetics, but examples thereof include vitamin B1, vitamin B2, vitamin B6, pyridoxine, pyridoxine hydrochloride, vitamin B 12, pantothenic acid, nicotinic acid, nicotinamide, folic acid, vitamin C, vitamin H, and the like, and salts thereof (thiamine hydrochloride, sodium ascorbate, and the like) or derivatives thereof (ascorbic acid-2-phosphate sodium salt, ascorbic acid-2-phosphate magnesium salt, and the like) are also included in the water-soluble vitamins which may be used in the present invention. The water-soluble vitamin may be obtained by a typical method such as a microbial transformation method, purification from a culture product of a microorganism, an enzymatic method or a chemical synthesis method.

Any oil-soluble vitamin may be used as long as it can be incorporated into cosmetics, but examples thereof include vitamin A, carotene, vitamin D2, vitamin D3, vitamin E (d1-alpha tocopherol, d-alpha tocopherol, and d-alpha tocopherol), and the like, and derivatives thereof (ascorbic palmitate, ascorbic stearate, ascorbic acid dipalmitate, dl-alpha tocopherol acetate, dl-alpha tocopherol vitamin E acetate, DL-pantothenyl alcohol, D-pantothenyl alcohol, pantothenyl ethyl ether, and the like) and the like are also included in the oil-soluble vitamins which are used in the present invention. The oil-soluble vitamin may be obtained by a typical method such as a microbial transformation method, purification from a culture product of a microorganism, an enzymatic method or a chemical synthesis method.

Any polymer peptide may be used as long as it can be blended with cosmetics, but examples thereof include collagen, hydrolyzed collagen, gelatin, elastin, hydrolyzed elastin, keratin and the like. The polymer peptide may be purified and obtained by a typical method such as purification from a culture solution of a microorganism, an enzymatic method or a chemical synthesis method, or typically may be purified from natural products such as the dermis of pigs, cows, and the like and silk fibers of silkworms and used.

Any polymeric polysaccharide may be used as long as it can be incorporated into cosmetics, but examples thereof include hydroxyethyl cellulose, xanthan gum, sodium hyaluronate, chondroitin sulfate, salts thereof (sodium salts, and the like), or the like. For example, chondroitin sulfate, salts thereof, or the like may typically be purified from mammals or fish and used.

Any sphingolipid may be used as long as it can be incorporated into cosmetics, but examples thereof include a ceramide, phytosphingosine, a glycosphingolipid and the like. The sphingolipids may typically be purified from mammals, fish, shellfish, yeast, plants, or the like by a typical method, or may be obtained by a chemical synthesis method.

Other ingredients typically incorporated into cosmetics may also be blended with the cosmetic composition of the present invention, if necessary, together with the essential ingredients.

Examples of other blended ingredients which may be added include oil and fat ingredients, a moisturizer, an emollient, a surfactant, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like.

Examples of the oil and fat ingredients include ester-based oils and fats, hydrocarbon-based oils and fats, silicone-based oils and fats, fluorine-based oils and fats, animal oils and fats, plant oils and fats, and the like.

Examples of the ester-based oils and fats include ester-based oils and fats such as glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linoleate, isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate, tri(capryl, capric acid)glyceryl, tri 2-ethylhexanoic acid trimethylol propane, triisostearic acid trimethyol propane, tetra 2-ethylhexanoic acid pentaerythritol, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linoleate, isopropyl isostearate, 2-ethylhexanoic acid cetostearyl, 2-ethylhexanoic acid stearyl, hexyl isostearate, ethylene glycol dioctanoate, ethylene glycol dioleate, propylene glycol dicaprylate, di(capryl, capric acid)propylene glycol, propylene glycol dicaprylate, neopentyl glycol dicaprate, neopentyl glycol dioctanoate, glyceryl tricaprylate, glyceryl triundecylate, glyceryl triisopalmitate, glyceryl triisostearate, octyldodecyl neopentanoate, isostearyl octanoate, octyl isononate, hexyldecyl neodecanoate, octyldodecyl neodecanoate, isocetyl isostearate, isostearyl isostearate, octyldecyl isostearate, polyglycerin ester oleate, polyglycerin ester isostearate, isocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stealoyl hydroxystearate, stearyl 12-stealoyl hydroxystearate, isostearyl 12-stealoyl hydroxystearate, or the like.

Examples of the hydrocarbon-based oils and fats include hydrocarbon-based oils and fats such as squalene, liquid paraffin, alpha-olefin oligomers, isoparaffin, sericin, paraffin, liquid isoparaffin, polybutene, microcrystalline wax, and Vaseline, or the like.

Examples of the silicone-based oils and fats include polymethyl silicone, methylphenyl silicone, methylcyclopolysiloxane, octamethyl polysiloxane, decamethyl polysiloxane, dodecamethyl cyclosiloxane, a dimethylsiloxane • methyl cetyloxysiloxane copolymer, a dimethylsiloxane • methyl stearoxysiloxane copolymer, alkyl-modified silicone oil, amino-modified silicone oil, and the like.

Examples of the fluorine-based oils and fats include perfluoropolyether, and the like.

Examples of the animal or plant oils and fats include animal or plant oils and fats such as avocado oil, almond oil, olive oil, sesame oil, rice bran oil, new flower oil, soybean oil, corn oil, rapeseed oil, apricot kernel oil, palm kernel oil, palm oil, castor oil, sunflower oil, grapeseed oil, cottonseed oil, palm oil, kukui nut oil, wheat germ oil, rice germ oil, shea butter, evening primrose oil, macadamia nut oil, meadow home oil, egg yolk oil, beef tallow, horse oil, mink oil, orange roughy oil, jojoba oil, candelilla wax, carnauba wax, liquid lanolin and hardened castor oil.

Examples of the moisturizer include a water-soluble low molecular weight moisturizer, a fat-soluble low molecular weight moisturizer, a water-soluble polymer, a fat-soluble polymer, and the like.

Examples of the water-soluble low molecular weight moisturizer include serine, glutamine, sorbitol, mannitol, pyrrolidone-sodium carboxylate, glycerin, propylene glycol, 1,3-butylene glycol, ethylene glycol, polyethylene glycol B (polymerization degree n=2 or more), polypropylene glycol (polymerization degree n=2 or more), polyglycerin B (polymerization degree n=2 or more), lactic acid, lactate, and the like.

Examples of the fat-soluble low molecular weight moisturizer include cholesterol, cholesterol ester, and the like.

Examples of the water-soluble polymer include a carboxyvinyl polymer, polyaspartate, tragacanth, xanthane gum, methyl cellulose, hydroxymethyl cellulose, hydroxyethyl cellulose, hydroxypropyl cellulose, carboxymethyl cellulose, water-soluble chitin, chitosan, dextrin, and the like.

Examples of the fat-soluble polymer include a polyvinylpyrrolidone • eicosene copolymer, a polyvinylpyrrolidone • hexadecene copolymer, nitrocellulose, dextrin fatty acid ester, high molecular weight silicone, and the like.

Examples of the emollient include long-chain acylglutamate cholesteryl ester, cholesteryl hydroxystearate, 12-hydroxystearic acid, stearic acid, rosin acid, lanolin fatty acid cholesteryl ester, and the like.

Examples of the surfactant include a nonionic surfactant, an anionic surfactant, a cationic surfactant, an amphoteric surfactant, and the like.

Examples of the nonionic surfactant include auto-emulsified glycerin monostearate, propylene glycol fatty acid ester, glycerin fatty acid ester, polyglycerin fatty acid ester, sorbitan fatty acid ester, polyoxyethylene (POE) sorbitan fatty acid ester, POE sorbitol fatty acid ester, POE glycerin fatty acid ester, POE alkyl ether, POE fatty acid ester, POE hardened castor oil, POE castor oil, a polyoxyethylene • polyoxypropylene (POE • POP) copolymer, POE • POP alkyl ether, polyether-denatured silicone, alkanolamide laurate, alkylamine oxide, hydrogenated soybean phospholipid, and the like.

Examples of the anionic surfactant include fatty acid soap, α-acylsulfonate, alkyl sulfonates, alkyl allyl sulfonates, alkyl naphthalene sulfonates, alkyl sulfates, POE alkyl ether sulfate, alkyl amide sulfates, alkyl phosphates, POE alkyl phosphates, alkylamide phosphates, alkyloyl alkyl taurine salts, N-acylamino acid salts, POE alkyl ether carboxylates, alkylsulfosuccinates, sodium alkylsulfoacetates, acylated hydrolyzed collagen peptide salts, perfluoroalkyl ester phosphate, and the like.

Examples of the cationic surfactant include alkyltrimethylammonium chloride, stearyltrimethylammonium chloride, stearyltrimethylammonium bromide, cetostearyltrimethylammonium chloride, distearyldimethylammonium chloride, stearyldimethylbenzylammonium chloride, behenyltrimethylammonium bromide, benzalkonium chloride, diethylaminoethylamide stearate, dimethylaminopropylamide stearate, lanolin derivative quaternary ammonium salts, and the like.

Examples of the amphoteric surfactant include amphoteric surfactants of the following types: carboxybetaines, amidebetaines, sulfobetaines, hydroxysulfobetaines, amidesulfobetaines, phosphobetaines, aminocarboxylates, imidazoline derivatives and amideamines.

Examples of the organic and inorganic pigments include inorganic pigments such as silicic acid, anhydrous silicic acid, magnesium silicate, talc, sericite, mica, kaolin, bengala, clay, bentonite, titanium-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine blue, chromium oxide, chromium hydroxide, carmine, and complexes thereof; organic pigments such as polyamide, polyester, polypropylene, polystyrene, polyurethane, vinyl resins, urea resins, phenol resins, fluororesins, silicone resins, acrylic resins, melamine resins, epoxy resins, polycarbonate resins, a divinyl benzene • styrene copolymer, silk powder, cellulose, CI pigment yellow and CI pigment orange; complex pigments of these inorganic pigments and organic pigments, and the like.

Examples of the organic powder include metal soaps such as calcium stearate; alkylphosphoric acid metal salts such as sodium zinc cetylphosphate, zinc laurylphosphate and calcium laurylphosphate; acylamino acid polyvalent metal salts such as N-lauroyl-β-alanine calcium, N-lauroyl-β-alanine zinc and N-lauroylglycine calcium; amidesulfonic acid polyvalent metal salts such as N-lauroyl-taurine calcium and N-palmitoyl-taurine calcium; N-acyl basic amino acids such as N-ε-lauroyl-L-lysine, N-ε-palmitoyllysine, N-α-palmitoylornithine, N-α-lauroylarginine and N-α-hardened beef tallow fatty acid acylarginine; N-acylpolypeptides such as N-lauroylglycylglycine; α-amino fatty acids such as α-aminocaprylic acid and α-aminolauric acid; polyethylene, polypropylene, nylon, polymethyl methacrylate, polystyrene, a divinyl benzene • styrene copolymer, ethylene tetrafluoride, and the like.

Examples of the ultraviolet absorbent include para-aminobenzoic acid, ethyl para-aminobenzoate, amyl para-aminobenzoate, octyl para-aminobenzoate, ethylene glycol salicylate, phenyl salicylate, octyl salicylate, benzyl salicylate, butylphenyl salicylate, homomenthyl salicylate, benzyl cinnamate, 2-ethoxyethyl para-methoxycinnamate, octyl para-methoxycinnamate, glyceryl mono(2-ethylhexanoate) dipara-methoxycinnamate, isopropyl para-methoxycinnamate, diisopropyl • diisopropylcinnamic acid ester mixtures, urocanic acid, ethyl urocanate, hydroxymethoxybenzophenone, hydroxymethoxybenzophenonesulfonic acid and salts thereof, dihydroxymethoxybenzophenone, sodium dihydroxymethoxybenzophenonedisulfonate, dihydroxybenzophenone, tetrahydroxybenzophenone, 4-tert -butyl-4'-methoxydibenzoylmethane, 2,4,6-trianilino-p -(carbo-2'-ethylhexyl-1'-oxy)-1,3,5-triazine, 2-(2-hydroxy-5-methylphenyl)benzotriazole, and the like.

Examples of the bactericide include hinokitiol, triclosan, trichlorohydroxydiphenyl ether, chlorhexidine gluconate, phenoxyethanol, resorcin, isopropylmethylphenol, azulene, salicylic acid, zinc pyrithione, benzalkonium chloride, photosensitizing dye No. 301, sodium mononitroguaiacol, undecylenic acid, and the like.

Examples of the antioxidant include butylhydroxyanisole, propyl gallate, erythorbic acid, and the like.

Examples of the pH adjuster include citric acid, sodium citrate, malic acid, sodium malate, fumaric acid, sodium fumarate, succinic acid, sodium succinate, sodium hydroxide, disodium hydrogen phosphate, and the like.

Examples of the alcohol include higher alcohols such as cetyl alcohol.

Further, the blended ingredients that may be added in addition to the aforementioned ingredients are not limited thereto, and any of the ingredients can be blended within a range that does not impair the purpose and effect of the present invention, but may be blended at 0.01 to 5 wt%, or 0.01 to 3 wt% based on the total weight.

When the formulation of the present invention is a lotion, a paste, a cream, or a gel, animal fiber, vegetable fiber, a wax, paraffin, starch, tragacanth, a cellulose derivative, polyethylene glycol, silicone, bentonite, silica, talc, zinc oxide, or the like may be used as the carrier ingredient.

When the formulation of the present invention is a powder or a spray, lactose, talc, silica, aluminum hydroxide, calcium silicate, or a polyamide powder may be used as the carrier ingredient, and in particular, when the formulation is a spray, the formulation may include a propellent such as chlorofluorohydrocarbon, propane/butane or dimethyl ether.

When the formulation of the present invention is a solution or an emulsion, a solvent, a solubilizer or an emulsifier is used as the carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol or fatty acid esters of sorbitan.

When the formulation of the present invention is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used as the carrier ingredient.

When the formulation of the present invention is a surfactant-containing cleanser, an aliphatic alcohol sulfate, an aliphatic alcohol ether sulfate, sulphosuccinic acid monoester, isethionate, an imidazolinium derivative, methyltaurate, sarcosinate, fatty acid amide ether sulfate, alkylamidobetaine, an aliphatic alcohol, fatty acid glyceride, fatty acid diethanolamide, a vegetable oil, a lanolin derivative, an ethoxylated glycerol fatty acid ester, or the like may be used as the carrier ingredient.

With respect to the terms used in the present invention, general terms currently and widely used are selected in consideration of function in the present invention, but the terms may vary according to an intention of a technician skilled in the art, a precedent, an advent of a new technology, and the like. Further, in specific cases, there is also a term arbitrarily chosen by the applicant, and in this case, the meanings thereof will be described in detail in the corresponding part of the Detailed Description of the present invention. Accordingly, the term used in the present invention should not be defined merely as a simple name of the term, but should be defined based on the meaning of the term and overall content of the present invention.

Terms such as first and second may be used to describe various constituent elements, but the constituent elements are not limited by the terms. The terms are used only to distinguish one constituent element from another constituent element. For example, without departing from the scope of the invention, a first constituent element may be called a second constituent element, and similarly, the second constituent element may be called the first constituent element. The term and/or includes a combination of a plurality of related listed items, or any item among the plurality of related listed items.

Throughout the specification of the present invention, when one part "includes (comprises)" one constituent element, unless otherwise specifically described, this does not mean that another constituent element is excluded, but means that another constituent element may be further included. Throughout the specification of the present invention, a term of a degree, such as "about" or "substantially", is used as meaning a corresponding numerical value or used as a meaning close to the numerical value when a natural manufacturing and a material tolerance error are presented in a described meaning, and is used to prevent an unconscientious infringer from illegally using disclosed contents including a numerical value illustrated as being accurate or absolute in order to help understanding of the present invention.

Throughout the specification of the present invention, the term "combination thereof' included in the Markush type expression means a mixture or combination of one or more selected from the group consisting of constituent elements described in the Markush type expression, and means including one or more selected from the group consisting of the above-described constituent elements.

Hereinafter, preferred examples for helping with understanding of the present invention will be suggested. However, the following examples are provided only so that the present invention may be more easily understood, and the content of the present invention is not limited by the following examples.

### [Examples]

### Example 1. Construction of three-dimensional skin organoids and characterization of organoids constructed by existing method

### Example 1-1. Culture of human induced pluripotent stem cells

An induced pluripotent stem cell line (iPSC; CMC3) derived from a healthy subject was cultured on a culture dish coated with vitronectin (ThermoFisher) without supporting cells in an Essential 8 (Gibco) culture solution supplemented with Y-27632 (10 µM). The culture solutions were exchanged every day, and subculture was performed using ReLeSR^{™} (Stem Cell Technology) in a cycle of about 5 days.

### Example 1-2. Method for differentiating three-dimensional skin organoid using human induced pluripotent stem cell line

A skin organoid was cultured based on the literature disclosing an existing skin organoid differentiation protocol using induced pluripotent stem cell lines (Lee, J. et al. Hair-bearing human skin generated entirely from pluripotent stem cells. Nature 582, 399-404(2020)). The induced pluripotent stem cell colony was separated into small cell populations using ReLeSR^{™} (Stem Cell Technology) and then separated into single cells using Accutase (ThermoFisher). For embryoid body formation, 1000 single cells were aliquoted into each well of a U-bottom low-attachment 96-well plate (Corning). Essential 8 (Gibco) supplemented with Y-27632 (20 µM) was used as an embryoid body culture solution. The single cells were cultured for 2 days until the embryoid body grew to a size of 300 to 500 µm. On day 2 of differentiation, the formed embryoid bodies were transferred to a culture solution including 2% Matrigel (Matrigel; Corning), 10 µM SB431542 (Tocris), 4 ng/ml FGF2 (PeproTech) and 15 ng/ml BMP4 (PeproTech) for non-neural ectoderm differentiation induction. From day 6, 200 ng/ml FGF2 and 50 µg/ml LDN193189 (Tocris) in the culture solution were added to the medium in order to induce cranial neural crest cells. Further, on day 14, the organoid was transferred to a 6-well plate (low-attachment 6-well plate; Corning) containing 3 ml of a medium for skin organoid maturation, and the skin organoid was cultured in an orbital shaker (65 rpm; Thermo Fisher) until the day of analysis. The media were exchanged every 3 days. The medium for skin organoid maturation includes 1X GlutaMax^{™} (Gibco), 0.5X B-27 minus vitamin 1 A (Gibco), 0.5X N2 (Gibco), a supplement, 0.1 mM 2-mercaptoethanol (Gibco) and 100 µg/ml normocin (Invitrogen) in an Advanced DMEM/F12 and Neurobasal medium at a ratio of 1:1. The skin organoid to be analyzed was photographed with an EVOS XL Core brightfield system microscope (Thermo Fisher) at x40 magnification.

As a result, as illustrated in FIG. 1, hair follicles were observed on day 60 to 70. A stratified epithelium was present in the inner layer of the organoid and surrounded by outer dermis and subcutaneous layers.

In addition, as illustrated in FIG. 2, the skin organoid grew to 5 mm or more in diameter in 140 days and included various pigments (top of FIG. 2) or albino hair follicles (bottom of FIG. 2). Organoids constructed by the existing skin organoid differentiation method (Lee et al. 2020) had the shape of round aggregates and had an inside-out morphology.

### Example 1-3. Fluorescence immunohistochemical staining

After cryosectioning skin organoids differentiated by the existing culture method (Lee et al. 2020), skin marker expression and a stratified structure were confirmed through fluorescence staining. First, the formed skin organoids were fixed with 4% PFA and then dehydrated in a 30% sucrose solution. After dehydration was completed, the organoids were frozen in a gelatin/sucrose solution, and slides were prepared by cryosectioning to a thickness of about 10 to 12 µm using a Cryostat instrument. The cryosections were blocked in a blocking buffer consisting of 5% normal chlorine serum, 0.5% bovine serum albumin, 0.25% fish gelatin and 0.3% TritonX-100 in PBS at room temperature for 1 hour. Primary antibodies were diluted in the blocking buffer and incubated at 4°C overnight. The sections were then washed three times with PBS and co-incubated with secondary antibodies in the blocking buffer at room temperature for 1 hour. The sections were washed three times with PBS, and nuclei were stained using 46-diamidino-2-phenylindole (DAPI). Finally, the sections were washed once with PBS and mounted with a fluorescent mounting medium (DAKO). Differentiation markers of the skin organoids cultured by the existing method were confirmed through fluorescence staining analysis, and formation and structure were confirmed by a cryosection slide staining method. The primary antibodies used are as follows: Anti-KRT5 (1:500; abeam; ab52635), Anti-KRT10 (1:100; Santa Cruz Biotechnology; sc-23877), Anti-KRT17 (1:100; Santa Cruz Biotechnology; sc-393091), Anti-Filaggrin (1:100; Santa Cruz Biotechnology; sc-66192), Anti-Vimentin vimentin (1:100; Santa Cruz Biotechnology; sc-6260), Anti-COL3A collagen 3 (1:100; Santa Cruz Biotechnology; sc-27124), Anti-Loricrin (1:500; abcam; ab85679), *Anti-Staphylococcus aureus* (1:100; Santa Cruz Biotechnology; sc-58038), Anti-PDGFRα (1:250; Cell Signaling Technology; 3164S), Anti-Melan A (1:250; abcam; ab51061), Anti-TSLP (1:250; abcam; ab47943), Anti-Lipid Tox (1:200; Invitrogen; H34477), Anti-Claudin4 (1:200; Invitrogen; 32-9400), Anti-KRT15 (1:200; Invitrogen MA5-11344), Anti-p63 (1:250; abcam; ab735), Anti-TFAP (1:50; Santa Cruz Biotechnology; sc-12726), Anti-COL2A1 (1:50; Millipore; ab261), Anti-Fibronectin (1:100; abeam; ab6328), Anti-S100α (1:50; Santa Cruz Biotechnology; sc-53438), Anti-E-cadherin (1:250; abcam; ab1416).

As illustrated in FIG. 3A, the formation and structure of epidermal progenitor cells (TFAP+KRT5+) and dermal progenitor cells (TFAP+PDGFRα+) were confirmed in skin organoids differentiated by the existing method.

As illustrated in FIG. 3B, the expression of Sox2+ dermal papilla cells was confirmed in skin organoid hair follicles cultured for 67 days and 87 days. The differentiation of hyaline cartilage was observed in differentiated skin organoids on day 67.

Furthermore, as illustrated in FIG. 3C, S100α+ chondrocytes and Coll2A1 were found to produce cartilage.

Next, stratified epidermal layer formation and structural timelines in skin organoids cultured by the existing method were investigated by a cryosection slide staining method.

As illustrated in FIG. 4, the expression of mature epidermal proteins was confirmed in skin organoids on days 33, 46, 67, 87, and 140. It could be seen that KRT5+ and KRT14+ basal layers were expressed in the skin organoids cultured for 33 days, and spinous keratinocytes expressing KRT10 were first observed in the surface epithelium on approximately day 46. A cornified epidermal layer expressing each of loricrin and filaggrin appeared, after 67 days of differentiation. That is, the existing skin organoid construction method shows the following differentiation expression characteristics. 1) The constructed organoids have the shape of round aggregates and have an inside-out morphology. A stratified epithelium is present in the inner layer of the organoid and surrounded by outer dermis and subcutaneous layers. 2) A mature cornified epidermal layer was observed in the innermost core of the 3D skin organoid. 3) Epidermal and dermal layer differentiation markers were confirmed, and the formation and structure of skin appendages such as hair follicle-constituting cells and dermal papilla cells and hyaline cartilage were confirmed by a cryosection slide staining method. 4) Final maturation of keratinization of the epithelial layer began on about day 70.

Therefore, the present inventors modified the existing three-dimensional skin organoid culture method to efficiently construct three-dimensionally stratified skin organoids and form skin organoids similar to real human skin. In the present invention, a three-dimensional air-liquid interface-skin organoid model (ALI-skin organoid) was constructed from human pluripotent stem cells by applying an air-liquid interface (ALI) culture method. The ALI culture method is a method widely used in skin tissue culture, and is close to the *in vivo* skin differentiation process by promoting the stratification of the epidermis and exposing the epidermal layer to air.

### Example 2. Construction of ALI-skin organoids by Wnt signaling pathway activation

The present inventors confirmed the effect of the Wnt signaling pathway on skin organoids through Wnt signaling pathway activation (0.5 to 8 µM Wnt agonist (CHIR-99021)) experiments.

First, the skin organoids were constructed based on the method of Example 1-2, but some steps were modified. From day 6, 200 ng/ml FGF2 and 50 µg/ml LDN193189 (Tocris) were added to the culture solution in order to induce cranial neural crest cells. In addition, 0.5 to 8 µM Wnt agonist (CHIR-99021) was added to the medium on day 6 for Wnt signaling pathway activation (see FIG. 5). On day 14 of culture, the organoid was transferred to a 6-well plate (low-attachment 6-well plate; Corning) containing 3 ml of a medium for skin organoid maturation, and the skin organoid was cultured in an orbital shaker (65 rpm; Thermo Fisher) until the day of analysis. The media were exchanged every 3 days. The skin organoid to be analyzed was photographed with an EVOS XL Core brightfield system microscope (Thermo Fisher) at x40 magnification.

As illustrated in FIGS. 6 and 7, as a result of adding 0.5 to 8 µM CHIR-99021, which is a Wnt agonist, to the culture solution on day 6 of differentiation, the sizes of the organoids were remarkably increased compared to the skin organoids generated by the existing method.

In addition, as illustrated in FIG. 7, in the group according to the existing culture method that was not treated with CHIR-99021 (CHIR(-)), cartilage differentiated in skin organoids on days 38, 65, and 85 (red circles indicate cartilage). However, it was confirmed that Wnt-activated skin organoids treated with CHIR-99021 increased in size and differentiated into spherical cysts in which cartilage was not formed, indicating that Wnt activation significantly suppresses the formation of cartilage, which is an unnecessary component in skin organoids.

### Example 3. Confirmation of expression of cartilage-related gene markers of ALI-skin organoids by Wnt signaling pathway activation

As a method for evaluating gene expression, cells were lysed and genes were extracted using a lysis buffer, and gene expression was quantified using RT-PCR. To evaluate gene expression, total RNA was isolated with an RNeasy kit (Qiagen, Cat. No. 74106) and cDNA was prepared according to the manufacturer's instructions using Reverse Transcriptase III (Thermo Fisher Scientific, Cat. No. 4368814). Real-time PCR was performed using SRBR Green Master Mix (Thermo Fisher Scientific, Cat. No. A25776) and detection was performed using a Step One Plus Real-time PCR system (Applied Biosystems). The expression of genes was normalized to the expression of glyceraldehyde-3-phosphate dehydrogenase (GAPDH). The degree of gene expression was evaluated using primers specific for COL2A, ACAN, and SOX9 mRNAs. The specific sequences of the primers are shown in the following Table 1.

**[Table 1]**

| mRNA | sequence | SEQ ID NO |
|---|---|---|
| COL2A | Forward-5'CATGAGGGCGCGGTAGAGAC3' | 1 |
| | Reverse-5 'TCCCTTTGGTCCTGGTTGCC3' | 2 |
| ACAN | Forward-5 'CTTCTCCGGAATGGAAACGTG3' | 3 |
| | Reverse-5 'ACATACCTCCTGGTCTATGTTACAG3' | 4 |
| SOX9 | Forward-5'GAAGCTCGCGGACCAGTACC3' | 5 |
| | Reverse-5 'CTGCCCGTTCTTCACCGACT3' | 6 |
| GAPDH | Forward-5'GTCAGTGGTGGACCTGACCT3' | 7 |
| | Reverse-5 'TGCTGTAGCCAAATTCGTTG3' | 8 |

As illustrated in FIG. 8, it was confirmed that in a CHIR-99021-treated skin organoid (CHIR (+)), the gene expression levels of the cartilage expression markers COL2A, ACAN, and SOX9 were remarkably reduced compared to the group not treated with CHIR-99021 (CHIR (-)).

### Example 4. Construction and characterization of skin organoids using air-liquid interface (ALI) culture method derived from human induced pluripotent stem cells

### Example 4-1. Construction of skin organoids using air-liquid interface (ALI) culture method

After the skin organoid of Example 2 was cultured for about 40 days to 100 days, the organoid was cut into 4 uniform sizes using Dumont #3 forceps (Fine Science Tools) and Spring Scissors (Fine Science Tools), and cultured by spreading the skin organoid on a collagen (Coming)-coated transwell culture insert (hole size 0.4 µm, 12-well plate; Coming), such that the dermal layer and the epidermal layer were exposed to the collagen side and air, respectively. The ALI-skin organoids were cultured with a skin maturation medium at the air-liquid interface in a 100% humidity condition incubator for an additional 3 to 4 weeks, and then cultured under dry conditions (a 0% humidity condition incubator) for maturation of the stratified epithelial layer for 2 to 6 days. The method for constructing an ALI-skin organoid is illustrated in FIG 9.

### Example 4-2. Characterization of skin organoids using air-liquid interface (ALI) culture method: histological structure confirmation

H&E staining and Oil Red O staining were performed to confirm the histological structure of a skin organoid. The H&E staining method is as follows. The organoid was fixed with 4% paraformalin, then dehydrated using alcohol, cleared with xylene, and then embedded in paraffin. The organoid was cryosectioned to a thickness of 12 µm to prepare a slide, and the slide was stained with Hematoxylin and Eosin, and then observed under an optical microscope (Olympus Ix70).

The Oil Red O staining method is as follows. The organoid was fixed with 4% paraformalin and then cryosectioned to a thickness of 12 µm to prepare a slide. The prepared slide was stained with an Oil O Red staining solution (0.5 g (Sigma Aldrich) in 100 ml isopropanol) for 15 minutes, stained with Hematoxylin, and then observed under an optical microscope (Olympus Ix70).

As illustrated in FIG 10A, it was observed that the ALI-skin organoid of the present invention not only showed a morphology more similar to real skin than the skin organoid cultured by the existing method, but also greatly improved the growth of hair follicles.

Furthermore, as illustrated in FIG 10B, the ALI-skin organoid of the present invention developed histologically into a continuous epithelial structure to form a mature stratified epithelial layer having a normal basket weave pattern on the surface, whereas existing whole skin organoids show a stratified epithelium, but the layers are inside the organoid and surrounded by outer dermis and subcutaneous layers, and thus showed an inside-out morphology different from real skin tissue. Therefore, it was confirmed that the ALI-skin organoid of the present invention is more suitable as a skin model representing real skin tissue.

In addition, as illustrated in FIG 11, it was confirmed that in the ALI-skin organoid constructed by the ALI culture method of the present invention, the subcutaneous fat layer was differentiated to the lower side of the dermal layer, similar to real skin, and it was observed that sebaceous glands developed around hair follicles also differentiated.

Furthermore, as illustrated in FIG 12, it could be seen that developed human hair follicle structures were formed in the ALI-skin organoid constructed by the ALI culture method of the present invention. It was also observed that hair follicle bulge stem cells, sebaceous glands, head bulbs, hair shafts, and the like were clearly expressed. This suggests that the ALI-skin organoid not only grows and expresses hair follicles reminiscent of the human skin structure, but also exhibits a remarkably improved morphology of hair follicles compared to existing organoids.

Further, as illustrated in FIG 13A, ALI-skin organoids cultured under dry conditions for 0, 2, 4, or 6 days for the maturation of skin keratinocytes of the present invention exhibited a morphology similar to that of skin tissue.

In addition, as illustrated in the H&E staining results in FIG 13B, differentiation of mature keratinocytes (red-stained areas) was observed in ALI-skin organoids cultured on days 4 and 6 under final dry conditions.

### Example 4-3. Characterization of skin organoid using air-liquid interface (ALI) culture method: fluorescence immunohistochemical staining

Using the fluorescence immunohistochemical staining method of Example 1-3, the expression of various skin-related factors was confirmed in the ALI-skin organoid of the present invention.

As illustrated in FIG 14, in the ALI-skin organoid of the present invention, KRT10, which is a marker for a mature epidermis and a mature stratum spinosum, was expressed, and the differentiation of loricrin and filaggrin, which are markers for the stratum corneum of the epidermis, was also observed.

Furthermore, as illustrated in FIG 15, in the ALI-skin organoid of the present invention, it could be confirmed that the expression of dermal papillae and melanocytes was observed in elongated hair follicles, and KRT15+ hair follicle bulge stem cells were also present. Further, it was observed that outer sheaths of hair shafts were expressed in the ALI-skin organoid. This suggests that the ALI-skin organoid of the invention exhibits improved hair follicle growth and expression reminiscent of the human skin structure.

In addition, as illustrated in FIG 16, in the ALI-skin organoid of the present invention, it was observed that the differentiation of loricrin and filaggrin, which are stratum corneum markers of the mature epidermis, began to appear in the ALI-skin organoid cultured on day 4 or 6 under dry conditions.

Furthermore, as illustrated in FIG 17, it was confirmed that collagen 3 (COL3) and vimentin (VMT), which are dermal layer markers, differentiated in the ALI-skin organoid cultured under dry conditions for 6 days, which is the final stage of the present invention.

Further, as illustrated in FIG 18, in the ALI-skin organoid cultured under dry conditions for 6 days, which is the final stage of the present invention, the expression of subcutaneous layer and sebaceous gland adipocytes could be confirmed by Lipid tox.

### Example 5. Atopic dermatitis modeling by infection with Staphylococcus aureus

### Example 5-1. Atopic dermatitis modeling by infection with Staphylococcus aureus: confirmation of histological structure

*Staphylococcus aureus,* which is a common cause of skin infections, is often found on the skin of atopic patients, but not on the skin of healthy persons. In order to model atopic dermatitis, it was checked whether *Staphylococcus aureus* penetrated the epidermal layer of the skin organoid when inoculated, and the colonization of epidermal *Staphylococcus aureus* was investigated using the ALI-skin organoid. Based on Example 4, ALI-skin organoids cultured for 3 to 4 weeks were infected using *S. aureus* strain ATCC 29213. A suspension was centrifuged at 100 × g for 10 minutes and resuspended in phosphate-buffered saline at a concentration of 1×10¹⁰ CFU/ml, 1×10⁹ CFU/ml, and 1×10⁸ CFU/ml calculated from the absorbance of the suspension at 600 nm. Next, 1 µl of the bacterial suspension was inoculated onto the ALI-skin organoid under 37°C and 5% CO₂ conditions for 24 hours. Thereafter, according to the fluorescence immunohistochemical staining method of Example 1-3, *Staphylococcus aureus,* a basal epithelial layer marker KRT5, a cell proliferation marker Ki67, stratum corneum markers filaggrin and loricrin, a basal epithelial layer marker KRT14, a stratum spinosum marker KRT10, and skin epidermal stem cell markers KRT15 and p63 were confirmed. Furthermore, cell death was confirmed through TUNEL analysis.

As illustrated in FIG 19, it was confirmed by immunostaining analysis that *Staphylococcus aureus* was detected in both the epidermis and dermis of the ALI-skin organoid inoculated with *Staphylococcus aureus.* It was confirmed that *Staphylococcus aureus* remarkably infects from the epidermal layer along the hair follicle to the dermal layer of the ALI-skin organoid, and causes colonization.

Further, as illustrated in FIG 20, it could be seen that the level of cell death was increased in the dermal layer of ALI-skin organoid inoculated with *Staphylococcus aureus,* and since the expression level of KRT5 was remarkably reduced, structural damage to the epidermal and dermal layers of the inoculated ALI-skin organoid was observed.

In addition, as illustrated in FIG 21, since the expression levels of filaggrin and loricrin in the epidermal layer of the ALI-skin organoid inoculated with *Staphylococcus aureus* were significantly reduced, it was observed that the skin barrier was damaged.

Furthermore, as illustrated in FIG 22, in the ALI-skin organoid inoculated with *Staphylococcus aureus,* since it was confirmed that KRT14, which is a basal epithelial layer marker, was significantly decreased while TSLP expression of atopic dermatitis keratinocytes was significantly increased, it was confirmed that symptoms of atopic dermatitis were well induced.

Further, as illustrated in FIG 23, it was confirmed that the expression levels of skin epidermal stem cell markers KRT15 and p63 were significantly decreased in the ALI-skin organoid inoculated with *Staphylococcus aureus.*

In addition, as illustrated in FIG 24, it was confirmed that the skin barrier aggregate protein markers Claudin 4 (CLDN4) and KRT5 were significantly reduced in the ALI-skin organoid inoculated with *Staphylococcus aureus.*

### Example 5-2. Modeling of atopic dermatitis by infection with Staphylococcus aureus: gene expression change analysis using RNA sequencing

An RNA sequencing analysis method was used in order to compare and evaluate the gene expression between ALI-skin organoids treated with 1×10⁶ CFU *Staphylococcus aureus* (n = 3) and ALI-skin organoids not treated (control; n = 3) according to the method of Example 5-1. First, genes were extracted using the gene extraction method of Example 3. A gene quality check, reverse transcription and sequencing were performed using the Illumina sequencing platform and protocol at Macrogen Inc. (Seoul, Korea). Differentially expressed genes were defined as those with > 2-fold changes in expression and adjusted P-values < 0.05. Subsequently, functional annotation and gene set enrichment analysis were performed using Gene Ontology and KEGG databases. Among the 46,427 genes identified, 26,458 genes with at least 0 fragment per kilobase of one million mapped lead transcripts were excluded, and 19,969 genes were to be processed for differentially expressed gene (DEG) analysis.

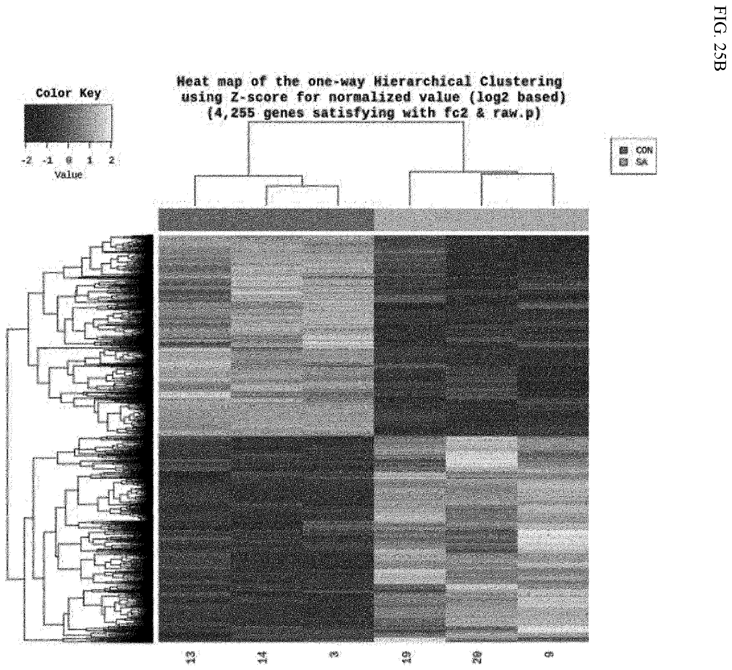

As illustrated in FIGS. 25A and 25B, 18 hours after surface inoculation of *Staphylococcus aureus* on the ALI-skin organoids, changes in gene expression were compared and analyzed by an RNA sequencing analysis method. Remarkable differences in the expression of numerous genes are shown between biological replicates between ALI-skin organoids treated with *Staphylococcus aureus* and untreated controls, and among 4,255 differentially expressed genes (DEGs), 2,162 genes were highly expressed and 2,093 genes were underexpressed by the inoculation of *Staphylococcus aureus.*

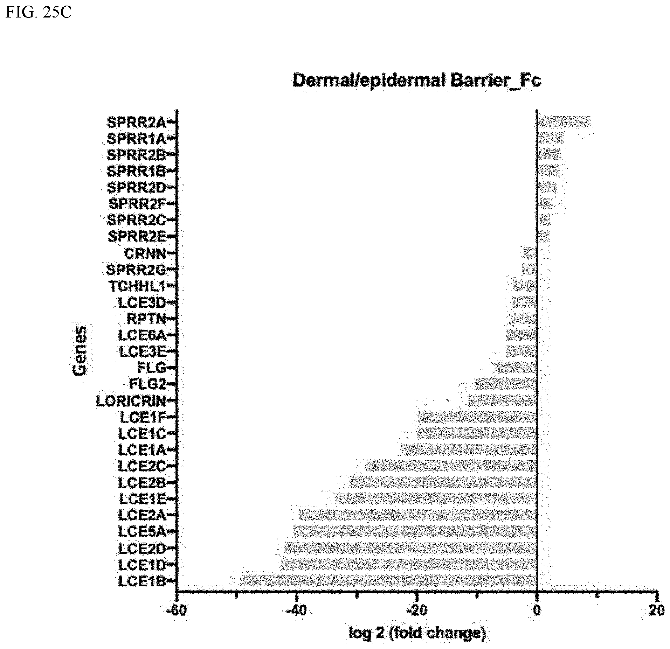

As illustrated in FIG 25C, in the ALI-skin organoids treated with *Staphylococcus aureus,* it was found that the expression of genes related to skin barrier function, such as filaggrin (FLG), late cornified envelope (LCE) and loricrin, was remarkably reduced compared to the control.

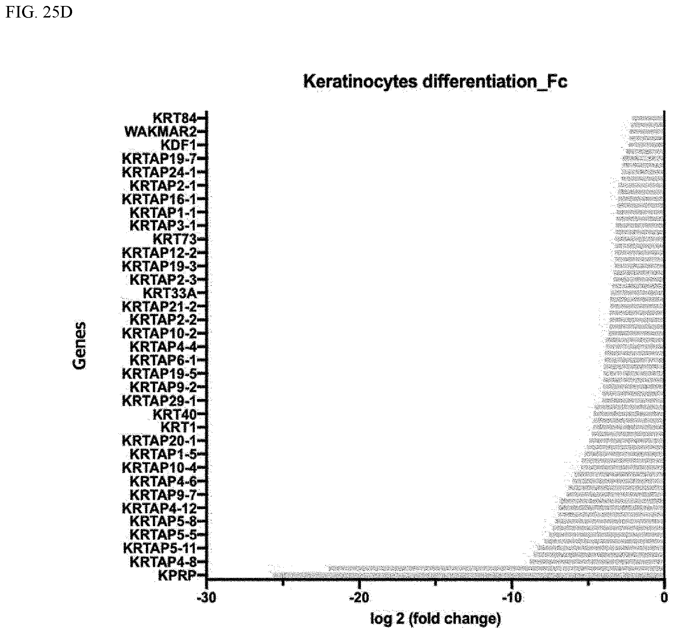

Furthermore, as illustrated in FIG 25D, as a result of RNA sequencing analysis, it was confirmed that the expression of keratinocyte differentiation-related genes such as KRT and KRTAP was remarkably lower in the ALI-skin organoids treated with *Staphylococcus aureus* than in the control.

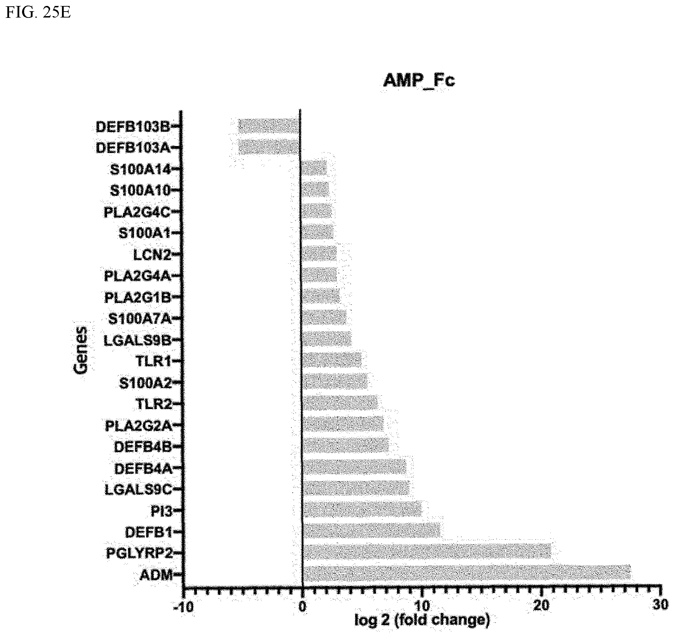

Further, the present inventors elucidated host cell responses to bacterial infection, including bacterial recognition, antimicrobial peptide (AMP) production and inflammatory signals. As illustrated in FIG 25E, in the RNA sequencing analysis results, it was confirmed that the expression of major antimicrobial peptide (AMP)-related genes such as defensin beta (Defb), peptidoglycan recognition protein 2 (Pglyrp2), toll-like receptors 1 and 2 (TLR1 and TLR2), galectin 9 (Lgals9), Lipocalin 2 (Lcn2) and S100 calcium-binding protein (S100A) was increased in the ALI-skin organoids treated with *Staphylococcus aureus* compared to the control.

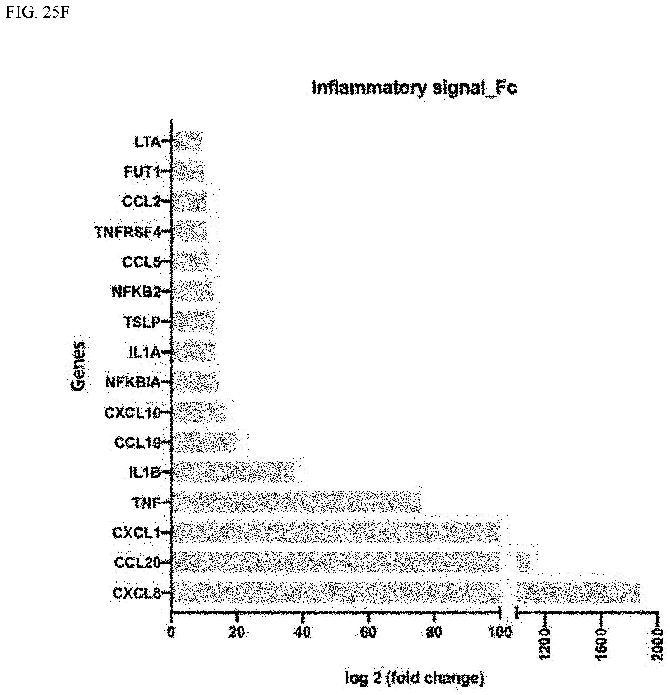

In addition, as illustrated in FIG 25F, it was confirmed that the expression of inflammatory cytokine-related genes such as CXC motif chemokine ligand (CXCL), CC motif chemokine ligand (CCL), TSLP, interleukin-1 (IL-1), and tumor necrosis factor (TNF) is significantly increased.

### Example 6. Confirmation of therapeutic effect of Cutibacterium acnes on ALI-skin organoids infected with Staphylococcus aureus

In the results shown in Example 5, it was confirmed that ALI-skin organoids infected with *Staphylococcus aureus* simulated the characteristics of atopic dermatitis well. Thus, in Example 6, it was investigated whether co-culturing a commensal microbiota found in healthy human skin could prevent or reduce the atopic dermatitis phenotype. *Cutibacterium acnes* is one of the most common commensal microorganisms found on healthy human skin. Thus, the therapeutic effect of a microbiome therapeutic agent using Cutibacterium acnes on the prevention of atopic dermatitis and skin barrier function was investigated.

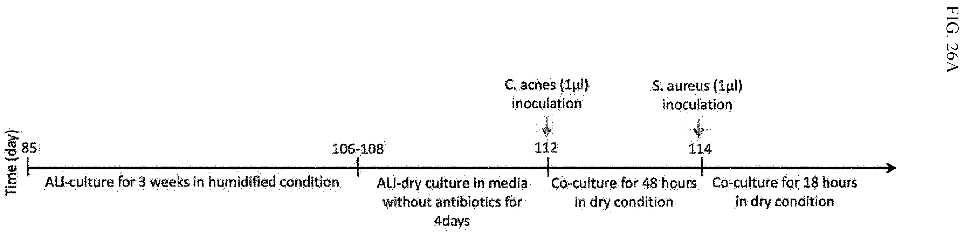

The ALI-skin organoids cultured for 3 to 4 weeks were cultured under dry conditions for 4 days based on Example 4, and then *Cutibacterium acnes* was suspended in phosphate buffered saline at a concentration of 1×10⁹ CFU/ml based on Example 5. Next, 1 µl of the bacterial suspension was inoculated onto the ALI-skin organoid under 37°C and 5% CO₂ conditions for 48 hours. After pretreatment with *Cutibacterium acnes* for 48 hours, Staphylococcus aureus was suspended in phosphate-buffered saline at a concentration of 1×10⁹ CFU/ml based on Example 5. ALI-skin organoids pretreated with *Cutibacterium acnes* were inoculated with 1 µl of the *Staphylococcus aureus* suspension under 37°C and 5% CO₂ conditions for 18 hours and cultured under dry conditions. Organoids were not inoculated (0, control), or inoculated with *Staphylococcus aureus* (SA) 1 × 10⁶ alone, Cutibacterium acnes (CA) 1 × 10⁶ CFU + SA 1 × 10⁶ CFU, or CA 1 × 10⁷ CFU + SA 1 × 10⁶ CFU. The ALI-skin organoid culture method and the *Cutibacterium acnes* inoculation method are illustrated in FIG 26A.

After inoculation, immunostaining for filaggrin, which is a key protein responsible for skin barrier function, was performed to check whether *Cutibacterium acnes* has an effect of protecting the skin barrier of ALI-skin organoids infected with *Staphylococcus aureus.* An epithelial layer marker KRT5 (CK5) and filaggrin, which is a key skin barrier protein, were analyzed and confirmed by the fluorescence immunohistochemical staining method of Example 1-3.

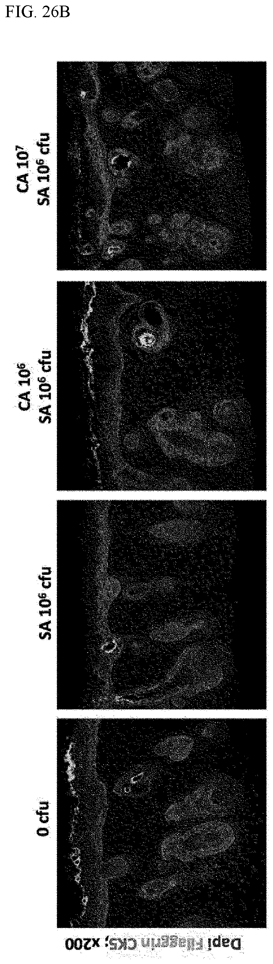

As illustrated in FIG 26B, filaggrin expression was remarkably reduced in ALI-skin organoids treated with 1×10⁶ Staphylococcus aureus alone. Conversely, ALI-skin organoids pretreated with 1×10⁶ CFU *Cutibacterium acnes* had significantly increased filaggrin expression levels compared to those treated with *Staphylococcus aureus* alone. These results indicate that *Cutibacterium acnes* diminishes the skin barrier disruption caused by infection with *Staphylococcus aureus* and provides a protective mechanism against the colonization of *Staphylococcus aureus.* Therefore, *Cutibacterium acnes,* which is one of the commensal microbiotas found in healthy human skin, has shown significant preventive and therapeutic effects against *Staphylococcus aureus*-mediated skin barrier disruption and cell damage, and thus, is expected to be used as a therapeutic agent for atopic dermatitis.

In summary, the skin organoids produced through the Wnt signaling pathway activation of the present invention remarkably increased the size of the organoid compared to the skin organoids produced by the existing method, and suppressed the formation of cartilage, which is an unnecessary component in skin organoids. Further, it was observed that ALI-skin organoids of the present invention formed skin cells, appendages, neural cells, adipocytes, and the like in similar structures to those in real skin and exhibited greatly improved follicle growth, compared to skin organoids constructed by existing culture methods. In addition, the atopic dermatitis organoid model of the present invention simulates the features of atopic dermatitis, such as a damaged skin barrier, an increase in epithelial cell-derived cytokines, downregulated expression of epidermal stem cells, and colonization of Staphylococcus aureus well, and thus is expected to be usefully used as a screening model for a therapeutic agent for atopic dermatitis.

Furthermore, using the atopic dermatitis model of the present invention, it was confirmed that *Cutibacterium acnes,* which is a human commensal bacterium, could be usefully used as a microbiome therapeutic agent by diminishing the skin barrier disruption due to infection with *Staphylococcus aureus* and providing a protective mechanism against colonization of *Staphylococcus aureus.* From the above results, it is expected that *Cutibacterium acnes* can be used for the treatment of atopic dermatitis.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

The present inventors confirmed that treatment with a Wnt agonist on day 6 not only remarkably increased the size of organoid, but also suppressed the formation of cartilage, which is an unnecessary component of skin organoids. It was observed that ALI-skin organoids of the present invention formed skin cells, appendages, neural cells, adipocytes, and the like in similar structures to those in real skin and exhibited greatly improved follicle growth, compared to skin organoids constructed by existing culture methods, and it was confirmed that organoids are formed in similar structures to those in real skin. In addition, as a result of treatment with Staphylococcus aureus to construct an atopic dermatitis model, a good simulation of the characteristics of atopic dermatitis, such as a damaged skin barrier, increased epithelium-derived cytokines, downregulated skin barrier function-related gene expression, downregulated keratinocyte differentiation-related gene expression, increased antimicrobial peptide gene expression, increased epidermal and dermal cell-derived cytokine gene expression, downregulated epidermal stem cell expression, causing Staphylococcus aureus colonization, and the like was made. Thus, the skin organoid of the present invention is expected to find advantageous applications as a real skin model and an atopic dermatitis model, and thus, has industrial applicability.

Meanwhile, using the atopic dermatitis model of the present invention, it was confirmed that *Cutibacterium acnes,* which is a beneficial human commensal bacterium as a microbiome therapeutic agent, diminished the skin barrier disruption due to infection with *Staphylococcus aureus,* and provided a protective mechanism against colonization of *Staphylococcus aureus.* Therefore, *Cutibacterium acnes* can be usefully used for the treatment of atopic dermatitis through significant preventive and therapeutic effects against *Staphylococcus aureus*-mediated skin barrier disruption and cell damage, and thus, has industrial applicability.

## Claims

1. A method for constructing a skin organoid from human pluripotent stem cells, the method comprising the following steps:
(a) culturing a pluripotent stem cell-derived organoid in the presence of a Wnt agonist;
(b) culturing a culture product of Step (a) in a medium for skin organoid maturation for 40 days or more; and
(c) cutting a culture product of Step (b) and culturing the cut culture product at an air-liquid interface.

2. The method of claim 1, wherein the Wnt agonist is CHIR-99021.

3. The method of claim 1, wherein the Wnt agonist is added on days 5 to 7 of culturing pluripotent stem cells.

4. The method of claim 1, wherein the medium for skin organoid maturation comprises one or more components selected from the group consisting of GlutaMax^{™}, 2-mercaptoethanol, B-27, N2, and normocin.

5. The method of claim 1, wherein the method comprises the following steps before performing Step (a):
(1) culturing an embryoid body of pluripotent stem cells;
(2) inducing the embryoid body to differentiate into non-neural ectoderm; and
(3) inducing the non-neural ectoderm to differentiate into cranial neural crest-like cells.

6. The method of claim 5, wherein Step (1) is performed in the presence of Y-27632.

7. The method of claim 5, wherein Step (2) is performed in the presence of SB431542, FGF2, and BMP4.

8. The method of claim 5, wherein Step (3) is performed in the presence of FGF2 and LDN193189.

9. The method of claim 1, wherein the culture of Step (a) is performed for 5 days to 14 days.

10. The method of claim 1, wherein the culture of Step (b) is performed for 40 days to 100 days.

11. The method of claim 1, wherein the culture of Step (c) is performed for 3 weeks to 4 weeks.

12. The method of claim 1, further comprising (d) culturing the culture product of Step (c) under dry conditions for 1 to 10 days.

13. The method of claim 12, wherein Step (d) is for stratum corneum maturation of the skin organoid.

14. The method of claim 1, wherein Step (c) comprises cutting the culture product of Step (b) into four uniform sizes, and
culturing the culture product of Step (b) such that the dermal layer and the epidermal layer on a collagen-coated transwell culture insert are exposed to the collagen side and air, respectively.

15. The method of claim 1, wherein the air-liquid interface culture of Step (c) is performed in a medium for skin organoid maturation.

16. The method of claim 1, wherein the skin organoid has suppressed cartilage formation.

17. The method of claim 1, wherein the skin organoid has a diameter of 2 to 20 mm.

18. The method of claim 1, wherein the skin organoid has a dermal layer and a subcutaneous fat layer located under the epidermal layer.

19. The method of claim 1, wherein the skin organoid expresses one or more selected from the group consisting of Keratin 5 (KRT5), KRT10, KRT15, KRT17, loricrin, filaggrin, SRY-Box Transcription Factor 2 (SOX2), and melan-A.

20. The method of claim 1, wherein the pluripotent stem cells are embryonic stem cells or induced pluripotent stem cells.

21. A method for constructing a skin organoid, the method comprising treating a skin organoid constructed by the method of claim 1 with *Staphylococcus aureus,* wherein the skin organoid is a skin simulation model of atopic dermatitis.

22. A skin organoid constructed by the method of claim 21, wherein the skin organoid is a skin simulation model of atopic dermatitis.

23. The skin organoid of claim 22, wherein the skin organoid has one or more features selected from the group consisting of the following features:
increased cell death in the skin dermal layer;
downregulated expression of one or more selected from the group consisting of KRT5, KRT10, filaggrin, loricrin, and Claudin 4, which are skin epidermal barrier-related markers;
decreased expression of KRT or KRTAP, which is a keratinocyte differentiation gene;
increased expression of one or more selected from the group consisting of β-defensin, peptidoglycan recognition protein 2 (PGLYRP2), toll-like receptor 1 (TLR1), TLR2, galectin 9 (LGALS9), lipocalin 2 (LCN2) and S 100 calcium binding protein (S 100A), which are antimicrobial peptide genes;
increased expression of one or more selected from the group consisting of CXC motif chemokine ligand (CXCL), C-C motif chemokine ligand (CCL), thymic stromal lymphopoietin (TSLP), interleukin-1 (IL-1), and tumor necrosis factor (TNF), which are epidermal or dermal cell-derived inflammatory cytokine genes;
downregulated expression of an epidermal stem cell marker KRT 15; and
colonization of Staphylococcus aureus.

24. A screening method for a therapeutic agent for atopic dermatitis, the method comprising treating the skin organoid of claim 22 with a candidate material for a therapeutic agent for atopic dermatitis.

25. A pharmaceutical composition for preventing or treating atopic dermatitis, comprising *Cutibacterium acnes* or a culture solution thereof as an active ingredient.

26. The pharmaceutical composition of claim 25, wherein the composition increases the expression of filaggrin.

27. A food composition for preventing or ameliorating atopic dermatitis, comprising *Cutibacterium acnes* or a culture solution thereof as an active ingredient.

28. A cosmetic composition for preventing or ameliorating atopic dermatitis, comprising *Cutibacterium acnes* or a culture solution thereof as an active ingredient.

29. A use of the skin organoid of claim 22 for screening a therapeutic agent for atopic dermatitis.

30. A method for preventing or treating atopic dermatitis, the method comprising administering a pharmaceutical composition comprising *Cutibacterium acnes* or a culture solution thereof as an active ingredient to a subject in need.

31. A use of a pharmaceutical composition comprising *Cutibacterium acnes* or a culture solution thereof as an active ingredient for preventing or treating atopic dermatitis.

32. A use of *Cutibacterium acnes* or a culture solution thereof for producing a drug for preventing or treating atopic dermatitis.
